# EUROPEAN PATENT APPLICATION

(11) **EP 4 653 425 A1**
(43) Date of publication of application: **26.11.2025**
(21) Application number: 24177129.4
(22) Date of filing: 21.05.2024
(51) Int. Cl.: C07D 237/26, C07B 59/00, C07D 257/08, C07D 487/04, C07D 491/04

(54) **METHOD FOR RAPIDLY PROVIDING PYRIDAZINES FROM TETRAZINES AND CIS-DIENOPHILES**

(71) Applicant: Tetrakit Technologies ApS, 2200 Copenhagen N (DK)
(72) Inventor: POULIE, Christian Bernard Matthijs, 2200 Copenhagen N (DK); BATTISTI, Umberto Maria, 2200 Copenhagen N (DK); HAUSER, Niklas Kent, 2200 Copenhagen N (DK); HERTH, Matthias Manfred, 2200 Copenhagen N (DK); JENSEN, Andreas Tue Ingemann, 2200 Copenhagen N (DK); MARTIN, Marcel, 2200 Copenhagen N (DK); SHALGUNOV, Vladimir, 2200 Copenhagen N (DK)
(74) Representative: Budde Schou A/S

(57) **Abstract**

The present invention relates to a method for providing pyridazines from 1,2,4,5-tetrazines and dionophile cyclenes having one or more rings wherein one ring is composed of at least 7 atoms, and having at least one unconjugated double bond in the cis-configuration via a one-pot-three-step reaction comprising *cis-trans* isomerization, ligation and oxidation, for providing pyridazines. The pyridazines can be used in therapy such as radiotherapy, diagnostics, imaging, and photochemistry methods.

## Description

### FIELD

The present invention relates to a method for providing pyridazines from 1,2,4,5-tetrazines and dienophile cyclenes having one or more rings wherein one ring is composed of at least 7 atoms, and having at least one unconjugated double bond in the cis-configuration via a one-pot-three-step reaction comprising *cis-trans* isomerization, ligation and oxidation, for providing pyridazines. The pyridazines can be used in therapy such as radiotherapy, diagnostics, imaging, and photochemistry methods.

### BACKGROUND

Labeled targeting vectors based on the tetrazine ligation can be used both for imaging purposes, such as diagnostics and other photochemistry imaging methods and in therapy. Such targeting vectors have for instance been labeled with radiolabels that can be applied in diagnostics and/or in therapy. The specific use depends on the identity of the radiolabeling used because different radionuclides provide for different purposes. The specific use moreover depends on the specific target that the vector is directed at. Several combinations of radiolabels and vectors are applied presently in diagnosis, therapy, theranostic and imaging. Different chemical entities connecting the radiolabeled entity with the target directed entity exists.

The term "tetrazine ligation" typically refers to a class of click reactions, where a tetrazine (Tz) and a *trans*-dienophile, typically a *trans*-cyclooctene (TCO) react in a fast, simple to use, versatile, chemo selective reaction, and give high product yields. These reactions have found application in a wide variety of research areas, including materials science, polymer chemistry, and pharmaceutical sciences.

Radiochemistry is one of the fields that showed the true potential of click chemistries as for example disclosed in Zeng et al, Journal of Nuclear Medicine, 54, 829-832, 2013. Essentially, the selectivity, ease, rapidity, and modularity of click ligations make them nearly ideally suited for the construction of radiotracers, a process that usually involves working with biomolecules in aqueous conditions with fast decaying radioisotopes.

The tetrazine ligation is characterized by the formation of covalent bonds between a 1,2,4,5-tetrazines and a *trans*-dienophile, such as *trans*-cyclooctene (TCO). The reaction is initiated by an inverse electron-demand Diels-Alder reaction, followed by a retro-Diels-Alder reaction, driven by the expulsion of N₂. The tetrazine ligation is among the fastest known chemical ligations, with second order rate constants up to 10⁶ M⁻¹s⁻¹ in acetonitrile at 25 °C. One major drawback of the conventional ligation of a Tz with a *trans*-dienophile such as TCO is that the ligation takes place without any regio-specificity. Moreover, the conventional ligation of a Tz with a *trans*-dienophile, such as TCO gives rise to several tautomeric forms. This means that conventional ligation of a Tz with for example TCO typically results in complex reaction mixtures of at least sixteen unique isomeric products (Scheme 1). Such mixtures of isomeric products cannot be applied directly for pharmaceutical purposes because the particular isomeric form of the compound influences the pharmacokinetics of the therapeutic agent and any potential toxicological effects of the individual isomeric forms cannot be distinguished.

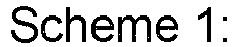

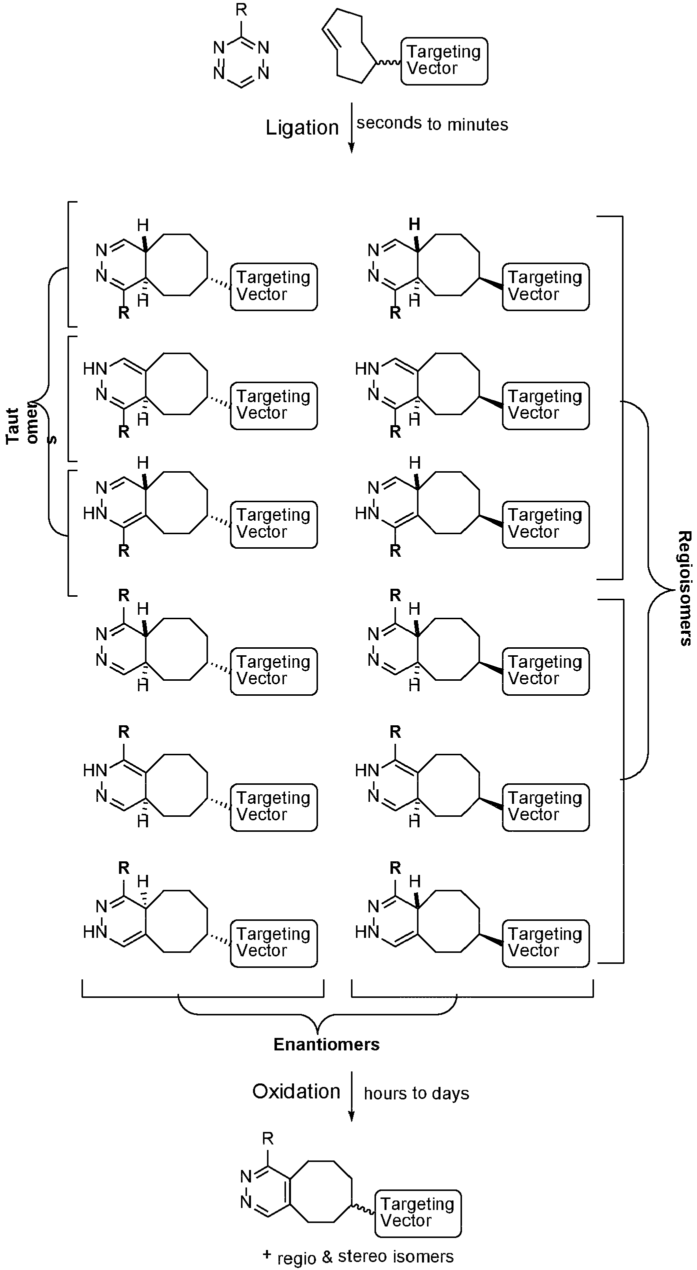

Scheme 1 is an overview of several isomeric forms after conventional tetrazine ligation with a *trans*-cyclooctene, inlcuding tautomers, enantiomers and regioisomers and subsequent slow auto-oxidation.

While the multiple dihydropyridazines that are obtained from conventional ligation may slowly auto-oxidize to the corresponding pyridazines over the course of several hours to several days, thereby slowly reducing the number of tautomeric forms of the products (WO2012/121746), the number of regio-isomeric and stereo-isomeric products are not reduced by this slow oxidation transformation. Means for speeding up the transformation from dihydropyridines to pyridazines have been described (Karaki Fumika et al, Tetrahedron, 97, 132411, 2021; Keinänen et al., ACS Medicinal Chemistry Letters, 7, 62-66, 2015), however, in none of reported cases was the oxidation completed within two hours, which is required if the pyridazines are to be used as radiopharmaceuticals. WO2017/059397, WO2020/242948, Syvänen et al., ACS Chemical Neuroscience, 11, 4460-4468, 2020, and WO2012/121746 discloses ligations between tetrazines and TCO's, which will inevitably provide several isomeric chemical entities. *cis*-Dienophiles, such as CCO can also react with a Tz Scheme 2 is an overview of the reactions between a tetrazine and a dienophile in the *trans-*configuration (2A) compared with reactions between a tetrazine and a dienophile in the *cis*-configuration (2B). As shown in Scheme 2A, the reaction between a tetrazine and a dienophile in the *cis*-configuration takes from several hours, up to several days and in addition also results the formation of complex reaction mixtures of at least sixteen unique isomeric products.

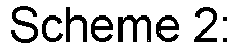

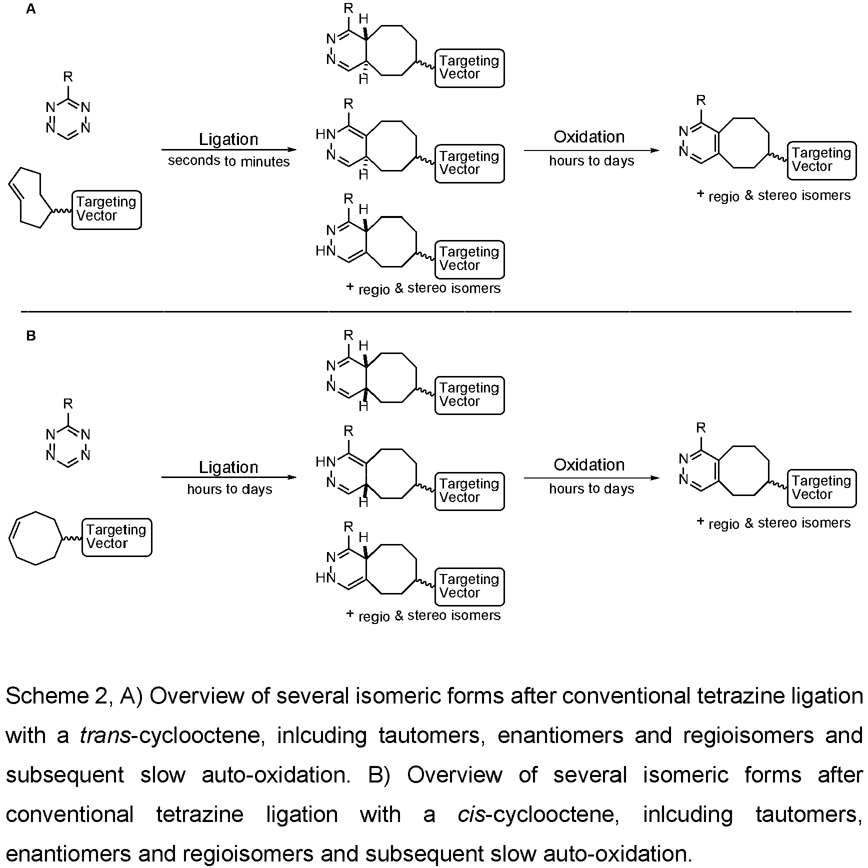

An other major obstacle is the chemical instability of *trans*-dienophiles, which upon various conditions, such as acidic envoirment, exposure to low intensity UV-light, or storage at ambient temperature readily isomerize to the much more stable *cis*-forms.

Alternatively, pyridazines can be prepared via the ligation of a Tz to a strained cyclic alkyne, however this reaction suffers from slow second order rate constants.

However, in order for click-chemistry such as the Tz-TCO ligation to furnish end-products that are viable in a regulatory and commercial context, it is desirable to reduce the number of ligation products produced. The presently available methods yields a large number of isomeric products that are impossible to separate, barring it from clinical translation due to toxicity concerns and unpredictable pharmacokinetics.

Moreover, a limitation to practical applicability of radiolabeled products is that the radiolabeled compound will have to be administered to a patient within a very limited time period from production for use in imaging, therapy or diagnostics. Therefore, any process that reduces the duration of the ligation step would be a great benefit, particularly when radioactive agents are used as labeling agents, diagnostic agents, therapeutic agents, theranostic agents or when used in combination with other agents.

It is generally the *trans* form of dienophiles, such as TCO is preferred in click chemistry reactions with tetrazines. Whereas the *cis* isomer is the more stable form, the ring-strain energies for cyclooctene being 16.7 and 7.4 kcal/mol, respectively, the *trans-*cyclene has a strained alkene bond, which makes it more reactive compared to the *cis* isomer. This higher reactivity makes it a better partner for the tetrazine in the click reaction. The same applies for the *cis-* and *trans*-forms, respectively, of also cyclenes with other numbers of C-atom member in the ring, such as cycloheptenes (CCH) and cyclononenes (CCN).

There are many routes to synthesize a *trans*-dienophile. A common way is the photoisomerization of *cis* to *trans*-form. Yet, the photoisomerization is in equilibrium between the two forms and favoring the *cis*-form, thus removal of the *trans*-isomer from the equilibrium is required to shift the equilibrium. Royzen et al. (J Am Chem Soc. 2008; 3760-3761) have proposed a procedure for the photochemical synthesis of TCO. In this method, TCO was produced via photoisomerization of *cis*-cyclooctene by exposure to UV light (254 nm) in a batch vessel. The reaction mixture was continuously passed through a bed of silver nitrate impregnated on silica gel. Subsequently, the TCO is selectively retained by silver nitrate and the remaining *cis-*cyclooctene-containing solution is recycled to the photochemistry batch vessel in order to drive the equilibrium towards the desired *trans*-form. The total yield was 66% and the total reaction time was 12 h.

Shahbazali et al. (AIChE J. 2021, e17067) suggested an integrated photo micro-flow adsorption method to produce *trans*-cyclooctene from its *cis*-isomer with the option to do that in recycle mode. There, the thermodynamic equilibrium was shifted by inserting an in-flow separation in a recycling flow mode, which makes better use of the given photoenergy.

When the dienophile is conjugated to another moiety such as a labeling agent, a pharmaceutical agent, or targeting vector, it is crucial to ensure a high degree of purity for the *trans*-isomer to maintain the integrity and effectiveness of the conjugate. Chromatography is normally used to separate a *trans*-dienophile being conjugated to another moiety from the equilibrium of the *cis* and *trans*-isomers. High-performance liquid chromatography (HPLC) or preparative chromatography can be particularly effective for purifying sensitive and valuable compounds, such as pharmaceutical agents or targeting vectors. It provides a high level of control over the separation process and allows for efficient collection of the desired isomer.

The present invention provides a method for providing labeled pyridazine products wherein the tetrazine and the more stable *cis*-form of a dienophile are mixed and wherein the specific reaction conditions applied provides for conversion of the *ci*s-dienophile to the corresponding *trans*-dienophile continuously as the *trans*-dienophile, upon forming, directly ligates with the tetrazine and thus is removed from the equilibrium, driving the isomerization to completion. Moreover, the applied conditions comprises direct oxidation of the clicked products (dihydropyridazines) so that the pyridazines are provided in a reduced complex mixture, depending on the specific *ci*s-dienophile and tetrazine used in the reaction. Accordingly, the present method comprises a one-pot-three-step reaction wherein isolation of the *trans*-dienophile prior to the click reaction is omitted. Additionally, the complexity of the inverse-electron demand Diels-Alder reaction is reduced, which eases the clinical translation and production of these compounds. The method advantageously enables radiolabeling, for example with ¹⁸F, ¹²³I, ¹²⁴I, ¹²⁵I or ¹³¹I, and ²¹¹At, of any tracer in unmatched efficiency and practicality.

### SUMMARY

The present invention provides in a first aspect a click-based method based on inverse electron demand Diels-Alder cycloaddition reactivity reaction between a tetrazine and a dienophile for providing pyridazines. The method applies to the conversion of a dienophile having one or more rings wherein one ring is composed of at least 7 atoms, and having at least one unconjugated double bond in the *cis-*configuration with a 1,2,4,5-tetrazine in order to obtain the corresponding pyridazines. The dienophile may be a mono- or bicyclic unsaturated hydrocarbon, or a mono- or bicyclic unsaturated heterocycle. Isomerisation of the *cis*-dienophile to the corresponding *trans*-dienophile and click-based ligation between the 1,2,4,5-tetrazine and the *trans*-dienophile together with rapid oxidation of the thus obtained dihydropyridazine mixture to pyridazine is achieved in a one-pot-three-step procedure by subjecting the mixture of 1,2,4,5-tetrazines and *cis*-dienophiles to a combination of acid and UV-light.

The method for providing pyridazines based on inverse electron demand Diels-Alder cycloaddition reactivity reaction between a tetrazine and a dienophile comprises:
a) Mixing:
   a dienophile selected from the group consisting of cyclenes having one or more rings wherein one ring is composed of at least 7 atoms, and having at least one unconjugated double bond in the *cis*-configuration; said dienophile being conjugated to a labeling agent and/or to a pharmaceutic agent, a diagnostic agent, or a therapeutic agent, or said dienophile being conjugated to a targeting vector; and
   a 1,2,4,5-tetrazine being conjugated to a labeling agent and/or a pharmaceutic agent, a diagnostic agent, or a therapeutic agent, or said 1,2,4,5-tetrazine being conjugated to or a targeting vector;

   in a solvent comprising at least 1% water,
   wherein when said dienophile is conjugated to a labeling agent and/or a pharmaceutic agent, a diagnostic agent, or a therapeutic agent, then said 1,2,4,5-tetrazine is conjugated to a targeting vector;
   and wherein when said dienophile is conjugated to a targeting vector, then said 1,2,4,5-tetrazine is conjugated to a labeling agent and/or a pharmaceutic agent, a diagnostic agent, or a therapeutic agent; and
b) subjecting the mixture obtained from step a) to a one-pot-three-step reaction at a temperature ranging from 15 °C to 50 °C by addition of an acid catalyst having a pKa of 5 or below in H₂O at 25 °C, at a concentration of 0.1 M - 5 M, and exposure to UV-light with a minimum UV irradiance of 0.5 mW/cm³.

The dienophile cyclene having one or more rings wherein one ring is composed of at least 7 atoms, and having at least one unconjugated double bond in the *cis-*configuration can be either a monocyclic unsaturated hydrocarbon, a bicyclic unsaturated hydrocarbon, a monocyclic unsaturated heterocycle, or a bicyclic unsaturated heterocycle.

The labeling agent can be any agent that is useful as a marker, an imaging agent, a therapeutic agent or a theranostic agent and includes radionuclides and fluorescent entities.

The targeting vector can be any suitable vector directed at a specific target and includes antibodies, nanobodies, polymers, nanomedicines, cells, proteins, peptides, and small molecules.

The products obtained by the present method are directed for use in theranostic, therapy, radiotherapy, diagnostic and imaging.

### BRIEF DESCRIPTION OF DRAWINGS

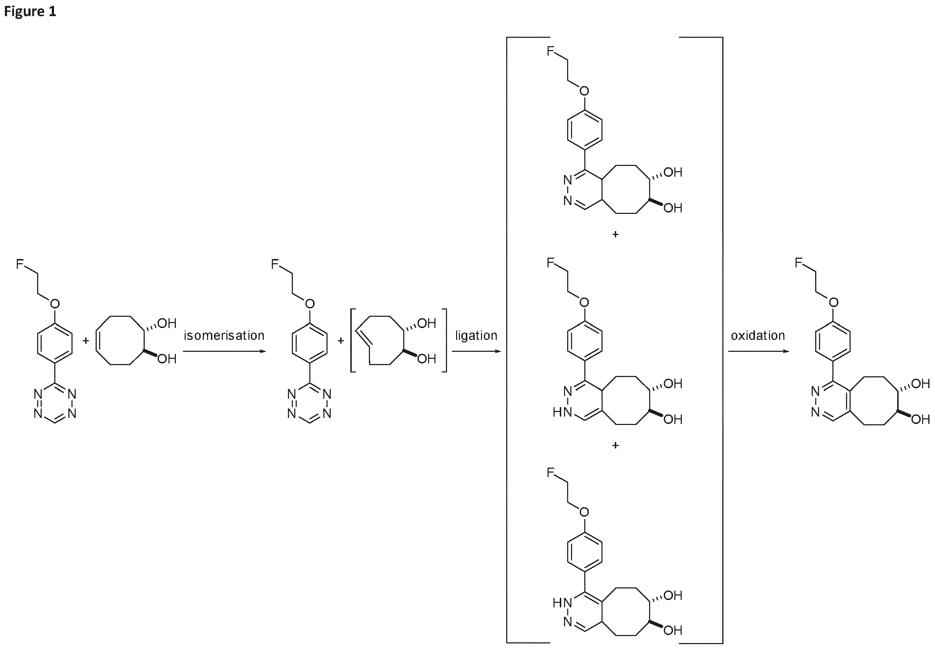

Figure 1: Reaction scheme showing an example of a tetrazine and CCO providing a pyridazine according to the present method.

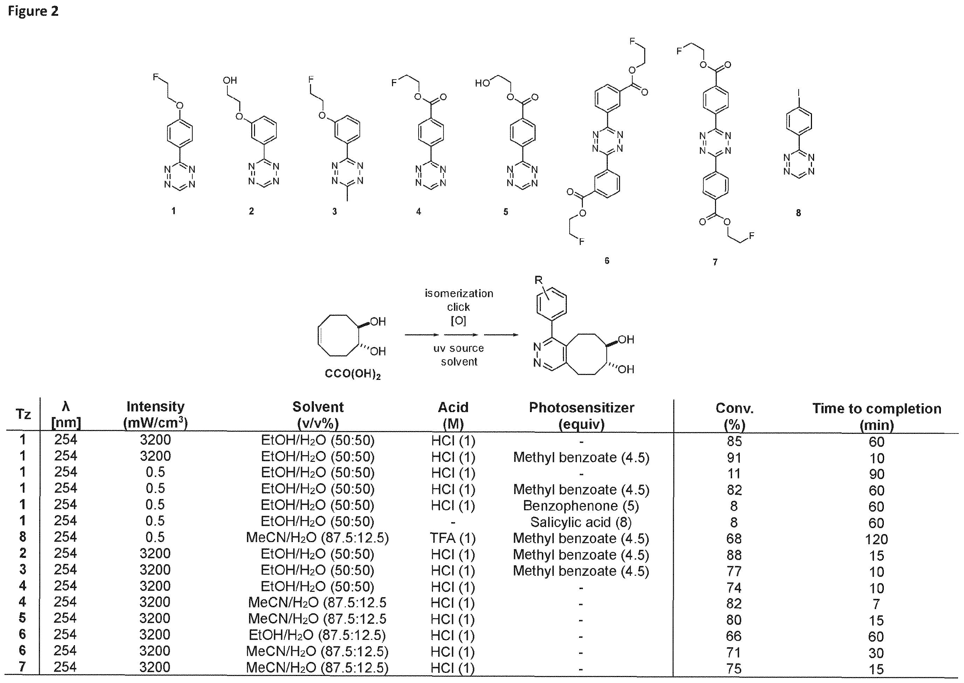

Figure 2: Structures of 8 tetrazines and CCO(OH)₂ applied herein and the reaction scheme for providing a pyridazine according to the present method and table showing the yield (%) (HPLC) and time to completion (min) in different combinations of tetrazines and CCO(OH2)₂ under different UV-sources, solvents, and acids, optionally with the addition of a photosensitizer.

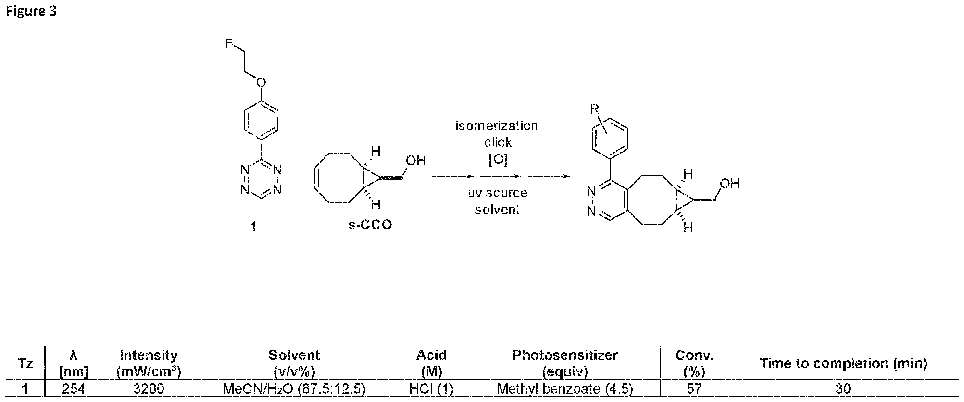

Figure 3: Structures of 1 tetrazine and s-CCO applied herein and the reaction scheme for providing a pyridazine according to the present method and table showing the yield (%) (HPLC) and time to completion (min) in different combinations of tetrazines and s-CCO under different UV-sources, solvents, and acids, optionally with the addition of a photosensitizer.

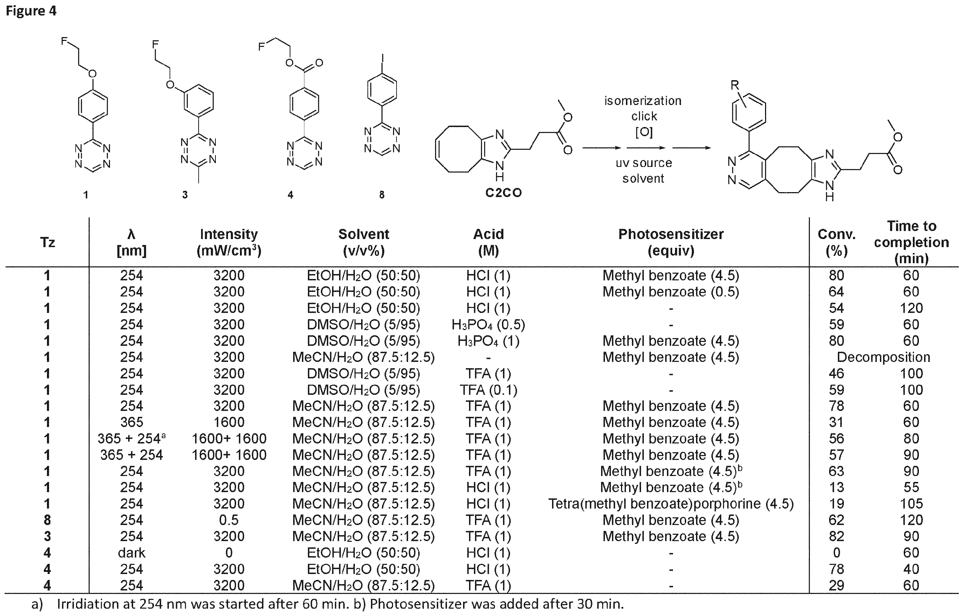

Figure 4: Structures of 4 tetrazines and C2CO applied herein and the reaction scheme for providing a pyridazine according to the present method and table showing the yield (%) (HPLC) and time to completion (min) in different combinations of tetrazines and C2CO under different UV-sources, solvents, and acids, optionally with the addition of a photosensitizer.

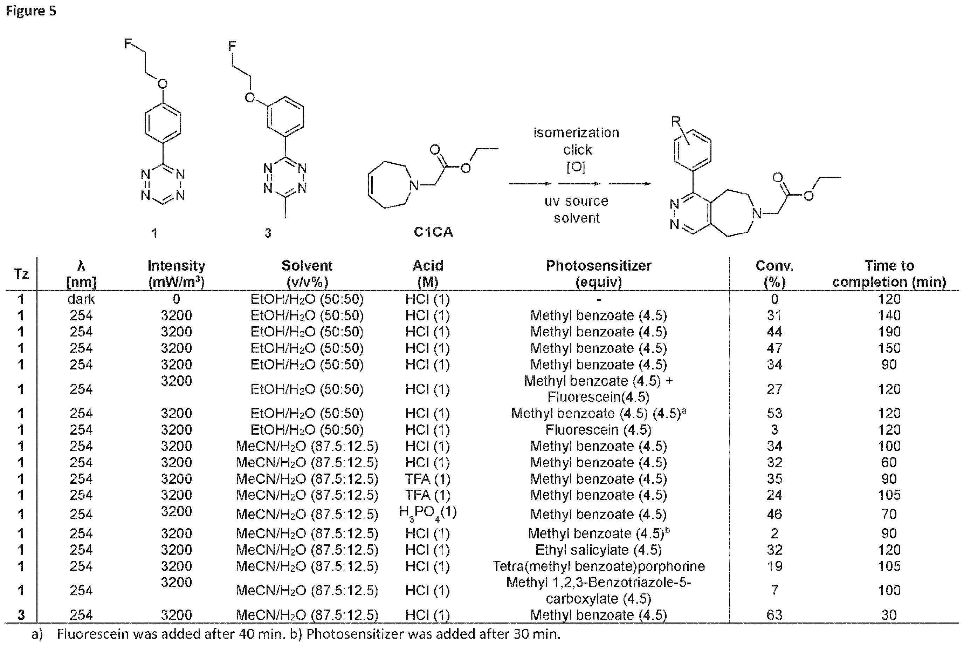

Figure 5: Structures of 3 tetrazines and C1CA applied herein and the reaction scheme for providing a pyridazine according to the present method and table showing the yield (%) (HPLC) and time to completion (min) in different combinations of tetrazines and C1CA under different UV-sources, solvents, and acids, optionally with the addition of a photosensitizer.

### DEFINITIONS

**Container** refers to the entity wherein chemical reactions, such as the three-step reaction of the present method takes place. The container is in the present context laboratory glassware such as vials. The vial can for example be made of borosilicate, quartz or other suitable material.

**A dienophile cyclene having one or more rings wherein one ring is composed of at least 7 atoms** means, that the dienophile is a cyclic structure and that the structure of the cyclene may comprise one or more rings. Further, at least one of the rings must be composed of at least 7 atoms, selected from carbon, nitrogen, oxygen, silicon, phosphor or sulfur atoms. Moreover, the dienophile cyclene must have **At least one unconjugated double bond in the *cis*-configuration** which refers to a molecular structure containing at least one single double bond, formed by sp2 hybridization, and the substituent groups attached to the atoms adjacent to the double bond are positioned on the same side of the molecule and that is not part of a conjugated system, i.e. next to another sp2 hybridized atom. This is also referred to as a **"*cis*-dienophile"** in the context of the present method. Conversely, a *"****trans-*dienophile"** is in the context of the present method a dienophile cyclene having one or more rings wherein one ring is composed of at least 7 atoms and having at least one unconjugated double bond in the *trans*-configuration. The trans-dienophile is obtained in the present method from the cis-dienophile in step b) of the method.

**Labeling agent** refers herein to an imaging agent that has been attached to a chemical entity having inverse electron demand Diels-Alder cycloaddition reactivity and being conjugated to a pharmaceutic agent, a diagnostic agent, or a therapeutic agent. The pharmaceutic agent, diagnostic agent, or therapeutic agent that the chemical entity is conjugated to is in some embodiments identical to the labeling agent. This may for instance be the case when the labeling agent is an agent that can be applied both as a label and as a therapeutic or diagnostic agent, such as the diagnostic imaging agent ¹⁸F. Labeling agents include radionuclides. However, since labeling of a diene or dienophile with a radionuclide will normally not provide 100% labeling efficiency with the radionuclide, some of the products labeled will inevitably be labeled with a stable isotope of the corresponding radionuclide element.

**Ligating** refers in the present context to a click reaction between the a first chemical entity having inverse electron demand Diels-Alder cycloaddition reactivity with a second chemical entity having complementary inverse electron demand Diels-Alder cycloaddition reactivity.

**"Solvent"** is in the present context an aqueous solution which means a mixture of at least 1% water and water miscible substances.

**"One-pot-three-step method" or "one-pot-three-step reaction"** means, that more than one chemical reactions that takes place simultaneously in the same container. In the present method, three chemical reactions takes place in step c), i.e. transformation of dienophile cyclenes having one or more rings wherein one ring is composed of at least 7 atoms, and having at least one unconjugated double bond in the *cis*-configuration to dienophile cyclenes having one or more rings wherein one ring is composed of at least 7 atoms, and having at least one unconjugated double bond in the *trans*-configuration; a click-ligation between the 1 ,2,4,5-tetrazine and the dienophile cyclene having one or more rings wherein one ring is composed of at least 7 atoms, and having at least one unconjugated double bond in the *trans*-configuration; and oxidation of the ligated dihydropyridazine into the corresponding pyridazine.

### DETAILED DESCRIPTION OF THE INVENTION

The present invention is based on the *in-situ* isomerization of a dienophile cyclene having one or more rings wherein one ring is composed of at least 7 atoms, and having at least one unconjugated double bond in the *cis*-configuration (herein also referred to as "the *cis*-dienophile" in relation to the present method) to the corresponding *trans*-dienophile, followed by a tetrazine ligation wherein a 1,2,4,5-tetrazine and the *trans*-dienophile are ligated, followed by a rapid oxidation of the corresponding click-product, i.e. mixture of dihydropyridazines, to the corresponding pyridazines, thereby reducing the complexity of the reaction products, which are bridging the labeled and the target directed entities.

The present invention provides a one-pot-three-step method for providing pyridazines from a mixture of a 1,2,4,5-tetrazine and a dienophile cyclene having one or more rings wherein one ring is composed of at least 7 atoms, and having at least one unconjugated double bond in the cis-configuration under specific conditions wherein three reaction steps takes place with high efficiency. The method comprises two steps: firstly, mixing a dienophile cyclene having one or more rings wherein one ring is composed of at least 7 atoms, and having at least one unconjugated double bond in the cis-configuration with a 1,2,4,5-tetrazine with complementary electron demand Diels-Alder cycloaddition reactivity and secondly, subjecting the mixture to UV-exposure, under acidic conditions. In the second step of the method, three chemical reactions occur rapidly and parallel in a one-pot-three-step reaction. Those three chemical reactions are 1) the conversion of the dienophile with at least one unconjugated double bond in the *cis*-configuration to the corresponding dienophile with at least one unconjugated double bond in the *trans*-configuration, 2) a click-reaction between the dienophile with at least one unconjugated double bond in the *trans*-configuration and the tetrazine, and 3) an oxidation of the dihydropyridazine mixture that arises from the click reaction to provide the corresponding pyridazine or a few pyridazine isomer forms.

The method comprises mixing a 1,2,4,5-tetrazine having inverse electron demand Diels-Alder cycloaddition reactivity and a dienophile cyclene having one or more rings wherein one ring is composed of at least 7 atoms, and having at least one unconjugated double bond in the *cis*-configuration having complementary inverse electron demand Diels-Alder cycloaddition reactivity in a solvent comprising at least 1% water. Either the 1,2,4,5-tetrazine or the dienophile cyclene having one or more rings wherein one ring is composed of at least 7 atoms, and having at least one unconjugated double bond in the *cis*-configuration is conjugated to a label whereas the other is conjugated to a targeting vector. The conversion of the dienophile cyclene having one or more rings wherein one ring is composed of at least 7 atoms, and having at least one unconjugated double bond in the *cis*-configuration to a dienophile cyclene having one or more rings wherein one ring is composed of at least 7 atoms, and having at least one unconjugated double bond in the *trans*-configuration, and the subsequent ligation between these entities is followed by a rapid acid catalyzed oxidation wherein all three chemical steps occurs subject to the same conditions. These conditions comprises addition of an acid catalyst having a pKa of 5 or below in H₂O at 25 °C, at a concentration of 0.1 M - 5 M, and exposure to UV-light with a minimum UV irradiance of 0.5 mW/cm³ at a temperature ranging from 15 °C to 50 °C. The advantage of the method is that all three reaction steps i.e. conversion from the *cis*-dienophile to the *trans*-dienophile, ligation of the tetrazine and the *trans-*dienophile, and the provision of one or a few isomeric forms of pyridazines are provided in a one-pot-three-step process subject to the same conditions. The pyridazines are provided within a minimum period of time and the complexity of the reaction mixture is highly reduced, thereby easing clinical translation and production costs.

Either the 1,2,4,5-tetrazine or the dienophile cyclene having one or more rings wherein one ring is composed of at least 7 atoms, and having at least one unconjugated double bond in the cis-configuration is conjugated to an agent of interest such as a pharmaceutic agent, a diagnostic agent, an imaging agent, or a therapeutic agent and labeled with a labeling agent, whereas the other entity is conjugated to a targeting vector of interest. Thus, when the dienophile cyclene having one or more rings wherein one ring is composed of at least 7 atoms, and having at least one unconjugated double bond in the *cis*-configuration is conjugated to a labeling agent and/or to a pharmaceutic agent, a diagnostic agent, or a therapeutic agent, then said 1,2,4,5-tetrazine is conjugated to a targeting vector. Conversely, when said dienophile is conjugated to a targeting vector, then said 1,2,4,5-tetrazine is conjugated to a labeling agent and/or a pharmaceutic agent, a diagnostic agent, or a therapeutic agent.

The UV-light exposure will trigger transformation of the dienophile cyclene in the *cis-*configuration into the *trans*-configuration and ligation between the tetrazine and the dienophile in the *trans*-configuration occurs spontaneously and rapidly via an inverse electron demand Diels-Alder cycloaddition reactivity, and accordingly, the tetrazine and the dienophile cyclene must have complementary inverse electron demand Diels-Alder cycloaddition reactivity.

In order to rapidly provide a labeled chemical entity or targeting vector, with improved homogeneity, that will bear a pyridazine, derived from a tetrazine, the resulting complex mixture of various dihydropyridazines will have to be oxidized.

It is shown herein that a suitable transformation of dienophile cyclene having one or more rings wherein one ring is composed of at least 7 atoms, and having at least one unconjugated double bond in the *cis*-configuration into its *trans*-configuration, ligation with the tetrazine and the and the oxidation of the thus obtained dihydropyridazines into pyridazine can be provided in a one-pot-three-step reaction when performed in a solvent comprising at least 1% water, and at a temperature ranging from 15 °C to 50 °C by addition of an acid catalyst, wherein the acid catalyst has a pKa of 5 or below in H₂O at 25 °C, at a concentration of 0.1 M - 5M in combination with UV-light with a minimum UV irradiance of 0,5 mW/cm³. A photosensitizer can optionally be included for further acceleration of either the isomerization or of the oxidation reaction or for acceleration of both reactions if suitable photosensitizers are present. Photosensitizers are dependent on the wavelength of the UV light applied and accordingly, the photosensitizer should be selected in accordance with the wavelength of the UV applied in the oxidation step. It is commonly known which photosensitizers that are suitable at a given wavelength and it is expected that the oxidation in step b) of the present method can be performed at UV-light wavelengths from 200 - 400 nm and the photosensitizer can be selected accordingly. In a preferred embodiment, the oxidation in step b) is performed at 200 - 300 nm, such as 200 - 350 nm, 250 - 350 nm, 250- 300 nm or such as at 254 nm. Preferred photosensitizers when UV-light at a wavelength of 200 - 350 nm such as at 254 nm is applied are benzoate based. Benzoate photosensitizers accelerate the *cis-trans* isomerization. Photosensitizers that accelerate the oxidation step can also be applied. Such photosensitizers are preferably fluorescein or porphyrin-based.

The photosensitizer can either be added during step b) of the method, or the photosensitizer can be an integrated part of the 1,2,4,5-tetrazine structure i.e. being conjugated to the 1,2,4,5-tetrazine structure or the photosensitizer can be an integrated part of the dienophile cyclene i.e. being conjugated to the dienophile cyclene having one or more rings wherein one ring is composed of at least 7 atoms, and having at least one unconjugated double bond in the *cis*-configuration.

The term acid applied in relation to catalyze the reaction in step b), refers to any molecule that has a proton with a pKa of at least 5 or lower, as measured in H₂O at 25 °C. Suitable acids have a pKa of -10 to 5, such as -8 to 3, -8 to 1, -6 to 1, -4 to 1, -2 to 1 or acids that have a pKa such as -10 to 2, -9 to 1, -8 to 0, -10 to -2, -10 to -4, -10 to -6 or such as acids that have a pKa of -8 to -10. The lower the pKa, the better in terms of reaction rate. Thus, in a preferred embodiment, the acids have a pKa of - 10 to 0 such as -10 to -8, -10 to -6, -10 to -4, -10 to -2 and -8 to 0.

Additionally, the concentration of acid in the solvent has to be 0.1 M - 5 M, such as 0.5-5 M, 1 - 5 M, 1.5-5 M, 2-5 M, 2.5-5 M, 3-5 M, 3.5-5 M, 4-5 M or 4.5-5 M. In a preferred embodiment, the concentration of acid in the solvent is 0.5 - 3 M, such as 0.5 - 2 M, such as 1 M.

Accordingly, the method for providing pyridazines based on inverse electron demand Diels-Alder cycloaddition reactivity reaction between a tetrazine and a dienophile comprises:
a) Mixing
   a dienophile selected from the group consisting of cyclenes having one or more rings wherein one ring is composed of at least 7 atoms, and having at least one unconjugated double bond in the cis-configuration; said dienophile being conjugated to a labeling agent and/or to a pharmaceutic agent, a diagnostic agent, or a therapeutic agent, or said dienophile being conjugated to a targeting vector; and
   a 1,2,4,5-tetrazine being conjugated to a labeling agent and/or to a pharmaceutic agent, a diagnostic agent, or a therapeutic agent, or said 1,2,4,5-tetrazine being conjugated to or a targeting vector;

   in a solvent comprising at least 1% water,
   wherein when said dienophile is conjugated to a labeling agent and/or to a pharmaceutic agent, a diagnostic agent, or a therapeutic agent, then said 1 ,2,4,5-tetrazine is conjugated to a targeting vector;
   and wherein when said dienophile is conjugated to a targeting vector, then said 1 ,2,4,5-tetrazine is conjugated to a labeling agent and/or a pharmaceutic agent, a diagnostic agent, or a therapeutic agent; and
b) subjecting the mixture obtained from step a) to a one-pot-three-step reaction at a temperature ranging from 15 °C to 50 °C by addition of an acid catalyst having a pKa of 5 or below in H₂O at 25 °C, at a concentration of 0.1 M - 5 M, and exposure to UV-light with a minimum UV irradiance of 0.5 mW/cm³,

The dienophile cyclene having at least one unconjugated double bond in the *cis-*configuration have preferably 7 to 12 atoms as ring members. In a preferred embodiment, the dienophile cyclene having at least one unconjugated double bond in the *cis*-configuration is selected from a C7 - C12 cyclene. Preferably, the dienophile cyclene having at least one unconjugated double bond in the *cis*-configuration is a C8 cyclene,

The dienophile cyclene having one or more rings wherein one ring is composed of at least 7 atoms, and having at least one unconjugated double bond in the *cis-*configuration can be either a monocyclic unsaturated hydrocarbon, a bicyclic unsaturated hydrocarbon, a monocyclic unsaturated heterocycle, or a bicyclic unsaturated heterocycle.

The pharmaceutic agent, diagnostic agent, or therapeutic agent that either the dienophile cyclene or the 1 ,2,4,5-tetrazine is conjugated to, is in some embodiments identical with the labeling agent. This may for instance be the case when the labeling agent is an agent that can be applied both as a label and as a therapeutic or diagnostic agent.

In some embodiments, the labeling agent is a radionuclide. Some radionuclides can be applied both in imaging, in diagnostics and/or in therapy and in the present examples, the same radionuclide have been applied as labeling agent as well as imaging or therapeutic agent. Labeling of a diene or dienophile with a radionuclide will normally not provide 100% labeling efficiency with the radionuclide, some of the products labeled will inevitably be labeled with a stable isotope of the corresponding radionuclide element.

The method enables labeling such as radiolabeling, for example with ¹⁸F, radioiodine (¹²³I, ¹²⁴I, ¹²⁵I or ¹³¹I) and ²¹¹At, of any targeting vector in unmatched efficiency and practicality. The ground-breaking nature of the method is the possibility of forming an end-product with improved homogeneity from the more stable cis-isomer of dienophiles. As shown herein, the present method may for some reactions provide formation of the end-product within a limited period of time such as 180 minutes, often within 60 minutes, such as within 10-20 minutes, and thereby easing clinical translation. In contrast, conventional tetrazine ligations result in multiple products and as a result need massive and unmanageable toxicological packages or tedious and time-consuming separation.

The pyridazine-containing targeting vectors obtainable by the method according to the present invention, can be applied for various purposes depending on the characteristics of the agent applied as a label. Labeling agents that are suitable for the method includes radiolabels and fluorescent labels.

In a preferred embodiment, the labeling agent applied in step a) in the method for providing a labeled pyridazine-containing targeting vector is a radionuclide or a stable isotope of a corresponding element. The characteristics and accordingly the use of the different radionuclides normally applied are well known in the art.

Radionuclide labeling agents and stable isotopes of a corresponding element that are suitable for use as a labeling agent in step a) in the method for providing a labeled pyridazine-containing targeting vector includes: ¹H, ²H, ³H, ¹¹C, ¹²C, ¹³C, ¹⁴C ¹³N, ¹⁴N, ¹⁵N ¹⁸F, ¹⁹F, ¹²³I, ¹²⁴I, ¹²⁵I, ¹²⁷I, ¹³¹I, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb,²¹²Pb, ²⁰⁹Bi, ²¹²Bi, ²¹³Bi, ³¹P, ³²P, ³³P, ³²S, ³⁵S ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh, ^{103m}Rh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ^{193mPt}, ^{195m}Pt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac. In a preferred embodiment, the radionuclide labeling agents is selected from the group consisting of: ¹¹C, ¹³N, ¹⁵O, ¹⁸F, ⁴³Sc, ⁴⁴Sc, ⁴⁵Ti, ⁵⁵Co, ⁶⁰Cu, ⁶¹Cu, ⁶⁴Cu, ⁶⁸Ga, ⁷⁶Br, ⁷²As, ⁸⁶Y, ⁸⁹Zr, ⁹⁰Y, ¹⁴⁹Tb, ¹⁵²Tb; and the stable isotopes of the corresponding element is selected from the group consisting of: ¹²C, ¹³C,¹⁴N, ¹⁵N, ¹⁶O, ¹⁷O, ¹⁸O, ¹⁹F, ⁴⁵Sc, ⁴⁶Ti, ⁴⁷Ti, ⁴⁸Ti, ⁴⁹Ti, ⁵⁰Ti, ⁵⁹Co, ⁶³Cu, ⁶⁵Cu, ⁶⁹Ga, ⁷¹Ga, ⁷⁵As, ⁷⁹Br, ⁸¹Br, ⁸⁹Y, ⁹⁰Zr, ⁹¹Zr, ⁹²Zr, ⁹⁴Zr, ¹⁵⁹Tb. These radionuclides and their stable isotopes of the corresponding elements are particularly useful in Positron Emission Tomography (PET).

In another preferred embodiment, the radionuclide labeling agents is selected from the group consisting of: ⁶⁴Cu, ⁶⁷Cu, ⁶⁷Ga, ¹¹¹In, ¹³¹I, ¹⁷⁷Lu, ¹⁸⁶Re, ²⁰¹Tl, ²¹²Pb, ²¹³Bi; and the stable isotope of the corresponding element is selected from the group consisting of: ⁶³Cu, ⁶⁵Cu, ⁶⁹Ga, ⁷¹Ga, ¹¹³In, ¹²⁷I, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi. These radionuclides and their stable isotopes of the corresponding elements are particularly useful in Single Photon Emission Computed Tomography (SPECT).

In another preferred embodiment, the radionuclide labeling agents is selected from the group consisting of: ³²P, ³³P, ⁴⁷Sc, ⁶⁴Cu, ⁶⁷Cu, ⁸⁹Sr, ⁹⁰Y, ¹⁶⁶Ho, ¹⁶¹Tb, ¹⁶⁵Dy, ¹⁷⁷Lu, ¹⁸⁶Re, ¹⁸⁸Re; and the stable isotope of the corresponding element is selected from the group consisting of: ³¹P, ⁴⁵Sc, ⁶³Cu, ⁶⁵Cu, ⁸⁴Sr, ⁸⁶Sr, ⁸⁷Sr, ⁸⁸Sr, ⁸⁹Y, ¹⁶⁵Ho, ¹⁵⁹Tb, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁷⁵Lu, ¹⁸⁵Re. These radionuclides are beta-particle emitters and these radionuclides along with their stable isotopes of the corresponding element are applied in therapy for instance in relation to the treatment of various tumorous diseases.

In another preferred embodiment, the radionuclide labeling agents is selected from the group consisting of: ¹⁴⁹Tb, ²¹²Pb, ²¹²Bi, ²¹³Bi, ²²⁷Th; and the stable isotope of the corresponding element is selected from the group consisting of: ¹⁵⁹Tb, ²⁰⁶Pb, ²⁰⁷Pb, ²⁰⁸Pb, ²⁰⁹Bi, ²³²Th. These radionuclides are alpha-particle emitters and these radionuclides along with their stable isotopes of the corresponding element are applied in therapy for instance in relation to the treatment of various tumorous diseases.

In another preferred embodiment, the radionuclide labeling agents is selected from the group consisting of: ⁵¹Cr, ⁵⁸mCo, ⁶⁴Cu, ⁶⁷Ga, ⁷³Se, ⁷⁵Se, ⁷⁷Br, ⁸⁰mBr, ⁹⁴Tc, ^{99m}Tc, ^{103m}Rh, ¹¹¹In, ¹¹⁴mIn, ¹¹⁵mIn, ¹¹⁹Sb, ¹²³I, ¹²⁴I, ¹²⁵I, ¹³⁵La, ¹⁶⁵Er, ^{193m}Pt, ^{195m}Pt; and the stable isotope of the corresponding element is selected from the group consisting of: ⁵²Cr, ⁵³Cr, ⁵⁴Cr, ⁵⁹Co, ⁶³Cu, ⁶⁵Cu, ⁶⁹Ga, ⁷¹Ga, ⁷⁴Se, ⁷⁶Se, ⁷⁷Se, ⁷⁸Se, ⁸⁰Se, ⁸²Se, ⁷⁹Br, ⁸¹Br, ¹⁰³Rh, ¹¹³In, ¹²¹Sb, ¹²³Sb, ¹²⁷I, ¹³⁸La, ¹³⁹La, ¹⁶²Er, ¹⁶⁴Er, ¹⁶⁶Er, ¹⁶⁷Er, ¹⁶⁸Er, ¹⁷⁰Er, ¹⁹²Pt, ¹⁹⁴Pt, ¹⁹⁵Pt, ¹⁹⁶Pt, ¹⁹⁸Pt. These radionuclides emit electrons via the Auger effect with low kinetic energy. These radionuclides along with their stable isotopes of the corresponding element are applied in Auger therapy for instance in relation to highly targeted treatment of various tumorous diseases.

In another preferred embodiment, the radionuclide labeling agents is selected from the group consisting of: ³H, ¹⁴C and ³⁵S and the stable isotope of the corresponding element is selected from the group consisting of: ¹H, ²H, ¹²C, ¹³C, ³²S. These radionuclides are applied to in vitro studies such as displacement and titration assays.

In another preferred embodiment, the radionuclide labeling agents is selected from the group consisting of: ¹¹C, ¹³N, ¹⁸F, ¹²³I, ¹²⁵I, ¹³¹I, or ²¹¹At; and the stable isotope of the corresponding element (when such a stable isotope of the element is obtainable) is selected from the group consisting of: ¹²C, ¹⁴N, ¹⁹F, ¹²⁷I. These radionuclides along with their stable isotopes of the corresponding element if available, are among the most frequently used radionuclides in therapy and imaging presently.

The targeting vector that is conjugated to either the 1,2,4,5-tetrazine or to the dienophile cyclene having one or more rings wherein one ring is composed of at least 7 atoms, and having at least one unconjugated double bond in the *cis*-configuration mentioned in step a) for providing a labeled pyridazine-containing targeting vector can be any kind of targeting vector that is suitable for use in therapy, imaging, or diagnostics. Such commonly used targeting vectors that are suitable in the present method include antibodies, nanobodies, polymers, nanomedicines, cells, proteins, peptides, and small molecules.

Commonly applied targeting vectors, that are suitable in the present method includes: peptides such as Octreotide, Octreotate, AE105; small molecules such as FAPI derivatives and PSMA derivatives.

In a preferred embodiment, the targeting vector applied in the method for providing a labeled pyridazine-containing targeting vector is selected from the group comprising: Octreotide derivatives, Octreotate derivatives, AE105 derivatives, FAPI derivatives and PSMA derivatives.

Step b) in the method for providing a pyridazine-containing targeting vectors is carried out at a certain temperature and time, by adding an acid and exposure to UV-light. These conditions ensure that the dienophile cyclene having one or more rings wherein one ring is composed of at least 7 atoms, and having at least one unconjugated double bond in the *cis*-configuration is transformed into the corresponding dienophile with the at least one unconjugated double bond in the *trans*-configuration which is then ligated to the tetrazine and that the resulting dihydropyridazine is oxidized into one or a few isomer forms of pyridazine

With the conditions applied in the present method, a sufficient amount of the pyridazines can be obtained within a short period of time, such as within a day. In many instances, the pyridazines can be provided within hours, such as within 180 minutes, such as from 0 - 160 minutes, from 0 - 120, from 0 - 90 minutes, from 0 - 60 minutes, from 0 - 50 minutes, from 0 - 40 minutes, from 0 - 30 minutes, from 0 - 20 minutes, from 0-10 minutes, or from 0 - 5 minutes.

The temperature in step b) is 15 °C - 50 °C, such as 15 °C - 45 °C, 15 °C - 40°C, 15 °C - 35 °C, 20 °C - 30 °C, or at approximately 20 °C - 25 °C. The preferred temperature is room temperature such as between 20 °C - 25 °C.

The *cis*/*trans*-isomerization are catalyzed photochemically by UV-light (with a minimum UV irradiance of 0.5 mW/cm³), whereas acid is required in addition for the oxidation. Accordingly, the acid may be added to the mixture obtained from step a) either before or after exposing the mixture to UV irradiance. Upon exposure, the mixture is exposed to UV-light, such as from 200 to 400 nm, 200 to 280 nm (UVC), 280 to 315 nm (UVB), 315 to 400 (UVA). Preferably 254 nm.

In some embodiments, the reaction in step b) is further catalyzed with the addition of one or more photosensitizers, such as fluorescein-based photosensitizer, aporphyrin-based photosensitizer or a benzoate-based photosensitizer, such as methyl benzoate. The photosensitizer may be added to the solvent or to the mixture in step a), or the photosensitizer may be part of the tetrazine structure, or the photosensitizer may be part of the dienophile structure. Alternatively, the one or more photosensitizers may be added during step b) either before or after exposure to UV. When a photosensitizer is added before exposure to UV, the mixture including the photosensitizer is exposed to UV-light, such as from 200 to 400 nm, 200 to 280 nm (UVC), 280 to 315 nm (UVB), 315 to 400 (UVA). Preferably at 254 nm.

In another preferred embodiment, the reaction in step b) is photochemically catalyzed by UVC-light with UV irradiance such as 0.5 to 12.500 mW/cm³, 500 to 10.000 mW/cm³, 500 to 5.000 mW/cm³, 500 to 3.750 mW/cm³, 1.250 to 3.750 mW/cm³, 2.500 to 3.750 mW/cm³, Preferably 3.200 mW/cm³.

Step b) is a one-pot-three-step reaction in the sense, that the three chemical reactions that takes place in step b) are performed in parallel in the same container. The foregoing step a) can optionally also be performed in the same container as step b), however, this is not a requirement of the method. In a preferred embodiment, step a) and b) is performed in the same container.

It was found that the solvent may influence on the reaction rate and thus on the time required for completing the reaction in step b) of the method. The more water that is present in the solvent, the faster the reaction rate. Accordingly, in a preferred embodiment, the solvent in step b) comprises 1 - 99% water, such as 20 - 95%, 30 - 95%, 40 - 95%, 50 - 95%, 60 - 95%, 70 - 95%, 80 - 95%, or 90 - 95% water, optionally with the addition of one or more photosensitizers.

It was also found that the material of the container, such as a vial, wherein the reaction in step b) is performed may influence the reaction rate and thus the time required for providing a sufficient amount of the pyridazine in step b) Thus, in a preferred embodiment, step b) is performed in a quartz container, optionally with the addition of one or more photosensitizers.

It was moreover found that the reaction time for the UV-light catalyzed reaction in step b) decreases when the reaction is performed in an open vial as compared to a closed vial. Thus, in a preferred embodiment, step b) is performed in an open container, optionally with the addition of one or more photosensitizers.

The intensity of the UV-light source may influence the reaction rate of the isomerization and the oxidation step. Thus, the reaction in step b) should be performed with UV-light with a minimum UV irradiance of 0.5 mW/cm³ . This will provide a sufficient amount of the oxidized pyridazine, within a day or even shorter, depending on the specific compounds applied. For many reactions, the oxidized pyridazines are provided within approximately 180 minutes.

The conventional ligation between a Tz and a *trans*-dienophile, such as TCO, will result in a complex mixture of different tautomers, regioisomers and enantiomers as schematically shown in Scheme 2A. This reaction typically occurs in seconds up to minutes. In contrast, the ligation between a Tz and a *cis*-dienophile, such as CCO, will occur over the span of several hours up to days and also result in a complex mixture of different tautomers, regioisomers and enantiomers as schematically shown in Scheme 2B. The subsequent auto-oxidation for both mixtures will additionally take place over the span of several hours up to days and reduce the complexity of the mixture by removing all tautomeric isomers, as schematically shown in Scheme 1.

Thus, multiple isomers would be formed from ligation of a tetrazine with any dienophile with at least one unconjugated double bond in the *trans*-configuration or with at least one double bond in the cis-configuration i.e. ligation of a tetrazine with either a TCO or a CCO. Multiple isomers (at least 12 isomers) can be formed in these reactions, which all have very similar polarities and are accordingly very difficult to separate for instance by HPLC.

In contrast, the method disclosed herein, allows for in situ transformation of a dienophile with at least one unconjugated double bond in the *cis*-configuration, such as a CCO, to the corresponding dienophile with at least one unconjugated double bond in the *trans*-configuration, such as a TCO, and subsequent rapid UV-catalyst reaction, which overall results in a fast conversion, which results in the reduction of complexity of the reaction mixture, i.e. increased homogeneity of the resulting pyridazines.

The starting entities to be ligated is a dienophile cyclene having one or more rings wherein one ring is composed of at least 7 atoms, and having at least one unconjugated double bond in the *cis*-configuration and 1,2,4,5-tetrazine.

Figure 1 shows and example of the present method wherein a fluor-labelled 1,2,4,5-tetrazine and CCO(OH)₂ is mixed in EtOH /2M HCl_{(aq.)} (1:1 v/v%) and exposed to UV-light (254 nm) providing three tautomeric forms of the F-labeled dihydropyridazine, after Z/E-isomerisation and ligation of the CCO(OH)₂, which following a UV-catalyzed oxidation results in a mixture of reduced complexity, i.e. increased homogeneity.

The first arrow indicates the isomerisation of CCO(OH)₂ to TCO(OH)₂. The next arrow indicates the click-ligation between the fluor-labelled 1,2,4,5-tetrazine and TCO(OH)₂. The third arrow indicates the UV-catalyzed oxidation of the ligated dihydropyridazines into corresponding pyridazine.

In a preferred embodiment, the dienophile cyclene having one or more rings wherein one ring is composed of at least 7 atoms, and having at least one unconjugated double bond in the *cis*-configuration in step a) is an isomer-free dienophile cyclene having one or more rings wherein one ring is composed of at least 7 atoms, and having at least one unconjugated double bond in the *cis*-configuration. Selecting an isomer-free dienophile with at least one unconjugated double bond in the *cis-*configuration will reduce the possible number to only a single-isomeric pyridazine structure, formed upon ligation with the 1,2,4,5-tetrazine.

In another preferred embodiment, the dienophile cyclene having one or more rings wherein one ring is composed of at least 7 atoms, and having at least one unconjugated double bond in the *cis*-configuration is an isomer-free dienophile cyclene having one or more rings wherein one ring is composed of at least 7 atoms, and having at least one unconjugated double bond in the cis-configuration and the 1,2,4,5-tetrazine is a symmetrical 1,2,4,5-tetrazine. This selection will result in a method wherein a single isomer form of the pyridazine is provided in step b).

The below structures are preferred dienophile cyclenes having one or more rings wherein one ring is composed of at least 7 atoms, and having at least one unconjugated double bond in the *cis*-configuration in step a) of the method for providing a labeled pyridazine-containing targeting vector: 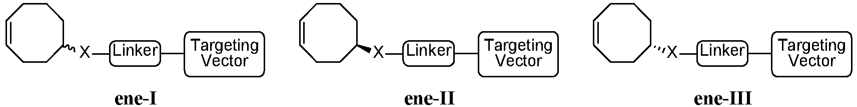 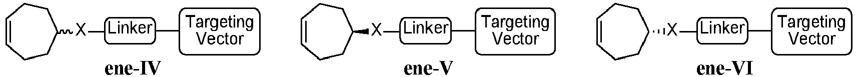 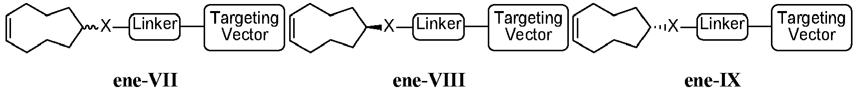 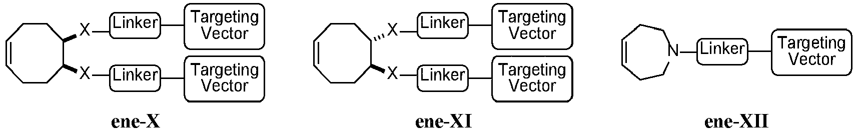 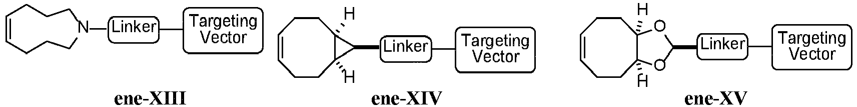 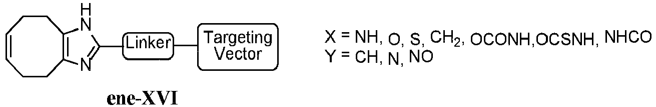
wherein the linker is selected from the group comprising: -(CH₂)ₙ- (CH₂)ₙNH, (CH₂)ₙCO, (CH₂)ₙO, (CH₂CH₂O)n, (CH₂CH₂O)nCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)nCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)nCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)nCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and wherein the targeting vector is an antibody, a nanobody, a polymer, a nanomedicine, a cell, a protein, a peptide, or a small molecule.

The following tetrazines of formula Tz1 are preferred 1,2,4,5-tetrazines in the present method: 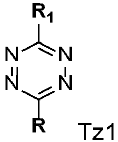
wherein R and R₁ are independently selected from -H, -Me or 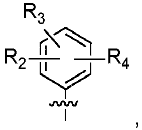 wherein the curly sign indicates the link to the tetrazine; and where R₂ is -H or (i) an isotope labeling agent directly connected to the aromatic ring; or (ii) an isotope labeling agent connected to the aromatic ring via a linker, said linker being selected from the group consisting of -(CH₂)ₙ, LO(CH₂)ₙ, -LNH(CH₂)ₙ, - LCONH(CH₂)ₙ, -LNHCO(CH₂)ₙ, where L is -(CH₂)ₘ or-(CH₂CH₂O)ₘ , where n and m are independently selected from 1-25; or (iii) an isotope labeling agent that is chelated through a chelator selected from: 1,4,7,10-tetraazacyclododecane-*N,N',N',N"*-tetraacetic acid (DOTA), *N,N'*-bis(2-hydroxy-5-(carboxyethyl)benzyl)ethylenediamine *N,N'*-diacetic acid (HBED-CC), 14,7-triazacyclononane-1,4,7-triacetic acid (NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15), 11,13-triene-3,6,9-triacetic acid (PCTA), *N*'-(5-acetyl (hydroxy)aminopentyl-N-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA) connected to the aromatic ring through a linker, said linker being selected from the group consisting of -(CH₂)ₙ, -LO(CH₂)ₙ, - LNH(CH₂)ₙ, -LCONH(CH₂)ₙ, -LNHCO(CH₂)ₙ, where L is -(CH₂)ₘ or -(CH₂CH₂O)ₘ, and n and m are independently selected from 1-25;
wherein, when R₂ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O, ¹⁶O, ¹⁷O, ¹⁸O ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac,
   and wherein R₃ and R₄ are independently selected from H or a moiety selected from the group consisting of a hydroxy group, a sulfonamide, a guanidyl group, a carboxyl group, a sulfonyl group, amine, a substituted amine with 1-25 polyethylene glycol unit(s), a -(O-CH₂-CH₂)ₙ--OCH₂-COOH, and n is selected from 1-25; or Methyl, Ethyl, Propyl, optionally substituted heteroaryl, and optionally substituted arylalkyl; wherein optionally substituted in relation to said substituted amine means one or more substituents selected from, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-25 polyethylene glycol unit(s), a -(O-CH₂-CH₂)ₙ-OCH₂-COOH, and n is selected from 1-25; or H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, and amine;

In preferred embodiments, the 1,2,4,5-tetrazine is a H-tetrazine, a methyl-tetrazine or a bisphenyl.

The below tetrazines are preferred tetrazines of Formula Tz1 for use in the present method for providing labeled pyridazine-containing targeting vector:

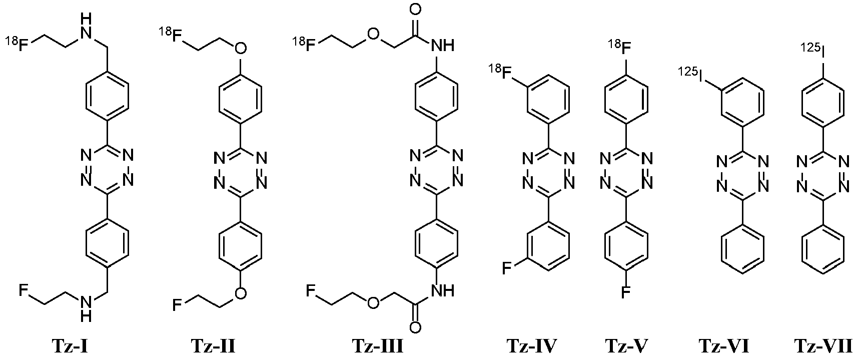

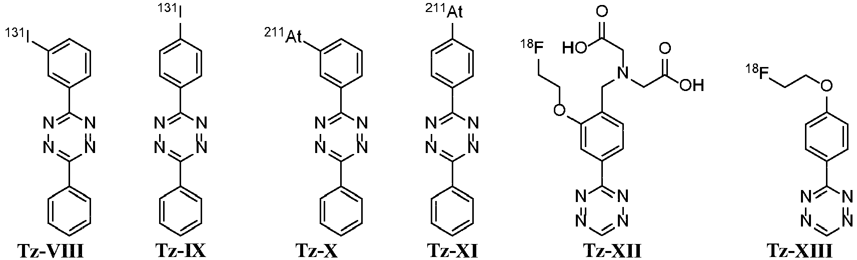

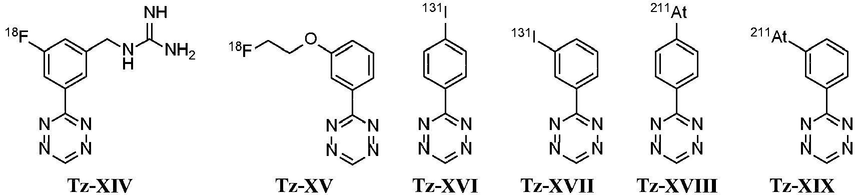

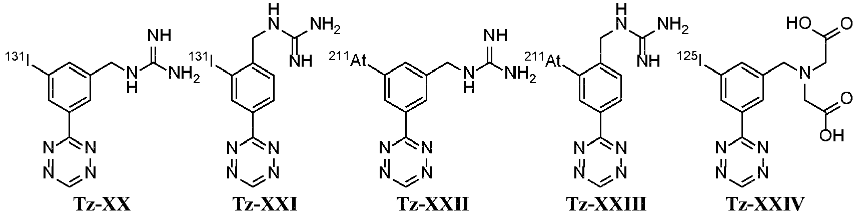

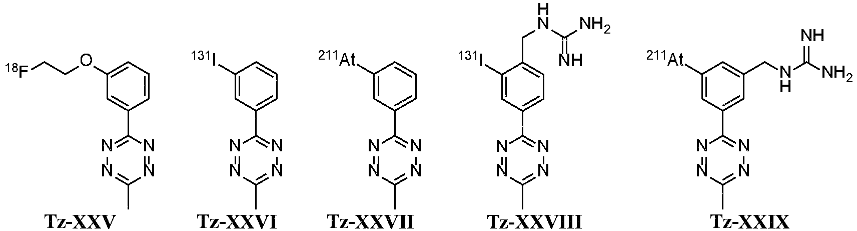

Tetrazine Tz-XX and Tz-XXI as shown above, comprises a photosensitizer in their structure. In a preferred embodiment, the 1,2,4,5-tetrazine of the present method comprises a photosensitizer and is selected from Tz-XX and Tz-XXI.

In another preferred embodiment, the 1,2,4,5-tetrazine is symmetrical substituted tetrazine wherein at least one of the symmetry planes pass through the nitrogen-nitrogen bonds of at least one tetrazine ring.

In a preferred embodiment, the 1,2,4,5-tetrazine is a symmetrical tetrazine selected from:

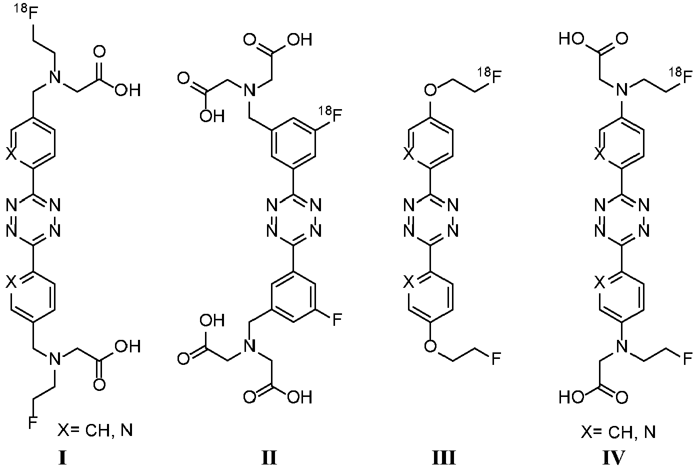

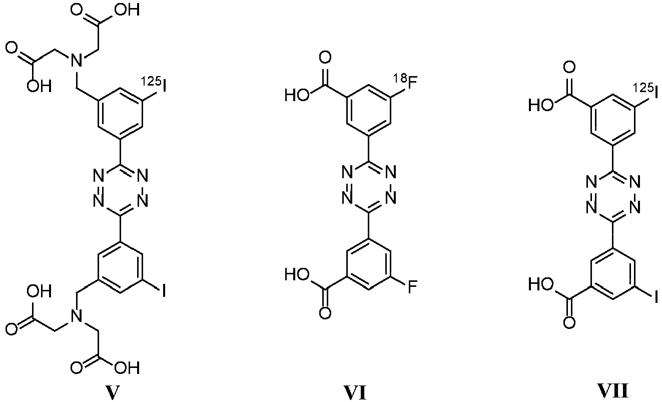

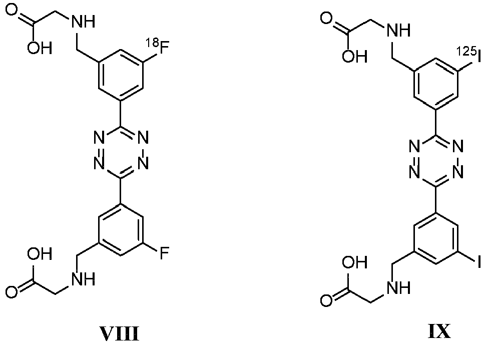

Step b) in the method for providing a pyridazine-containing targeting vector is a three-step reaction wherein the dienophile cyclene with at least one unconjugated double bond in the *cis*-configuration is transformed to the corresponding dienophile cyclene with at least one unconjugated double bond in the *trans*-configuration and ligation of the tetrazine and the dienophile in the *trans*-configuration takes place. Even though auto-oxidation of the ligated entity targeting vector would eventually occur spontaneously in the absence of the conditions in step b), this process is extremely slow and can last from several hours up to several days. Step b) in the method provides a fast way for reducing the number of different isomeric dihydropyridazines in the mixture.

The labeled pyridazine-containing targeting vector provided by the method can be used in therapy, radiotherapy, theranostics, diagnostics, or imaging, depending on the labeling agent, or the pharmaceutical agent, or imaging agent or therapeutic agent and on the targeting vector.

Preferably, the targeting vector is coupled to the linker via a nitrogen on the targeting vector, such as via an amide bond or a carbamate bond or an urea bond. Alternatively, the targeting vector is preferable coupled to the linker via a carbonyl on the targeting vector, such as via an amide bond.

In a preferred embodiment, the labeled pyridazine-containing targeting vector is used in therapy.

In another preferred embodiment, the labeled pyridazine-containing targeting vector is used in radiotherapy.

In another preferred embodiment, the labeled pyridazine-containing targeting vector is used in theranostics.

In another preferred embodiment, the labeled pyridazine-containing targeting vector is used in diagnostics.

In another preferred embodiment, the labeled pyridazine-containing targeting vector is used in imaging.

The following Examples describes (1) the synthesis of 1,2,4,5-tetrazines and dienophile cyclenes having one or more rings wherein one ring is composed of at least 7 atoms, and having at least one unconjugated double bond in the *cis-*configuration representative for use in the present method for providing a labeled pyridazine-containing targeting vector and (2) measurements of yield and time to completion of the and one-pot-three-step reaction taking place in step b) of the present method.

### EXAMPLES

### General

All reagents and solvents were dried prior to use according to standard methods. Commercial reagents were used without further purification. Analytical TLC was performed using silica gel 60 F254 (Merck) with detection by UV absorption and/or by charring following immersion in a 7% ethanolic solution of sulfuric acid or KMnO₄-solution (1.5 g of KMnO₄, 10 g K₂CO₃, and 1.25 mL 10% NaOH in 200 mL water). Purification of compounds was carried out by column chromatography on silica gel (40-60 µm, 60 Å) or employing a CombiFlash NextGen 300+ (Teledyne ISCO). ¹H and ¹³C NMR spectra were recorded on Brucker (400 and 600 MHz instruments), using Chloroform-*d*, Methanol-*d*₄ or DMSO-*d*₆ as deuterated solvent and with the residual solvent as the internal reference. For all NMR experiences the deuterated solvent signal was used as the internal lock. Chemical shifts are reported in δ parts per million (ppm). Coupling constants (*J* values) are given in Hertz (Hz). Multiplicities of ¹H NMR signals are reported as follows: s, singlet; d, doublet; dd, doublet of doublets; ddd, doublet of doublets of doublets; dt, doublet of triplets; t, triplet; q, quartet; m, multiplet; br, broad signal. NMR spectra of all compounds are reprocessed in MestReNova software (version 12.0.22023) from original FID's files. Mass spectra analysis was performed using MS-Acquity-A: Waters Acquity UPLC with QDa-detector. Purification by preparative HPLC was performed on Agilent 1260 infinity system, column SymmetryPrep-C18, 17 mL/min H₂O-MeCN gradient 50-100% 15 min with 0.1% trifluoroacetic acid. All final compounds were >95% pure as determined by analytical HPLC. Analytical HPLC method: (Thermo Fisher^{®} UltiMate 3000) with a C-18 column (Luna^{®} 5u C18(2) 100Å, 150 × 4.6 mm), eluents: A: H₂O with 0.1% TFA, B: MeCN with 0.1% TFA. Gradient from 100% A -> 100% B over 15minutes, back to 100% A over 4 minutes, flow rate 1.5 mL/min. Detection by UV-absorption at λ = 254 nm on a UVD 170U detector.

### Example 1

### Synthesis of tetrazines and their precursors

### Example 1.1

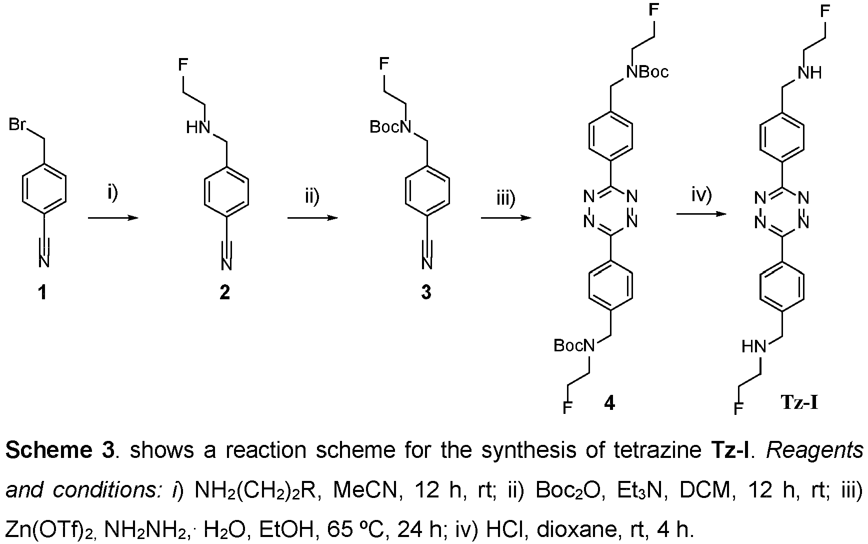

### 4-(((2-Fluoroethyl)amino)methyl)benzonitrile (2)

To a solution of 4-(bromomethyl)benzonitrile (0.78 g, 4.00 mmol) in CH₃CN (40 mL) was added K₂CO₃ (0.33 g, 24.0 mmol) and 2-fluoroethylamine hydrochloride (0.16 g, 16.0 mmol). The mixture was stirred at room temperature overnight. The solvent was removed under reduced pressure, and the residue was diluted with water (20 mL), extracted with EtOAc. The combined organic layer was washed with brine, dried over MgSO₄, filtered and concentrated under reduced pressure. The crude product was purified by flash column chromatography using EtOAc (Heptane/EtOAc 50/50) in heptane to afford 0.54 g (76%) of the desired product as a colorless oil. R*_{f}* = 0.24 (Heptane/EtOAc 40/60). ¹H NMR (400 MHz, CDCl₃) *δ* 7.55 (d, *J* = 8.2 Hz, 2H), 7.40 (d, *J* = 8.0 Hz, 2H), 4.63 - 4.48 (m, 1H), 4.47 - 4.37 (m, 1H), 3.84 (s, 2H), 2.93 -2.84 (m, 1H), 2.84 -2.72 (m, 1H), 1.65 (s, 1H). ¹³C NMR (101 MHz, CDCl₃) *δ* 145.6, 132.3, 128.6, 118.9, 110.9, 83.5 (d, *J* = 165.5 Hz), 53.1, 49.1 (d, *J* = 19.7 Hz).

### tert-Butyl 4-cyanobenzyl(2-fluoroethyl)carbamate (3)

To a solution of 4-(((2-fluoroethyl)amino)methyl)benzonitrile (540 mg, 3.03 mmol) and Et₃N (1.27 mL, 9.09 mmol) in CH₂Cl₂ (40 mL) was added Boc₂O (790 mg, 3.63 mmol) and the mixture was stirred at room temperature for 12 h. The solution was washed with water and saturated K₂CO₃ solution, dried over Na₂SO₄, filtered and concentrated under reduced pressure. The crude product was purified by flash column chromatography using (Heptane/EtOAc 70/30) to afford 0.710 g (84%) of the desired product as a colorless oil (mixture of rotamers). R*_{f}* = 0.42 (Heptane/EtOAc 80/20). ¹H NMR (400 MHz, CDCl₃) *δ* 7.55 (d, *J* = 7.8 Hz, 2H), 7.27 (d, *J* = 7.8 Hz, 2H), 4.79 - 4.10 (m, 4H), 3.62 - 3.28 (m, 2H), 1.96 - 1.05 (m, 9H). ¹³C NMR (101 MHz, CDCl₃) *δ* 155.4, 144.2, 143.8, 132.4, 128.1, 127.5, 118.7, 111.1, 83.2 (d, *J* = 168.2 Hz), 82.7 (d, *J* = 170.5 Hz), 52.1, 51.2, 47.7, 28.3.

### Di-tert-butyl (((1,2,4,5-tetrazine-3,6-diyl)bis(4,1-phenylene))bis(methylene))bis((2-fluoroethyl)carbamate) (4)

To a suspension of *tert*-butyl 4-cyanobenzyl(2-fluoroethyl)carbamate (1.1 gr, 3.95 mmol) and Zn(OTf)₂ (0.72 gr, 1.98 mmol) in EtOH (30 mL) was added hydrazine monohydrate (3.83 mL, 79 mmol). The mixture was allowed to stir at 70 °C for 22 hours, and when the reaction is completed, is cooled at room temperature. The volatiles were removed under reduced pressure and the residue solubilized in EtOH (40 mL). A solution of NaNO₂ (5.52 g, 80.00 mmol ) in water (20 mL) was added to the crude reaction followed by dropwise addition of HCl (2M) until gas evolution ceased and a pH of 2-3 was achieved producing a red mixture. The crude reaction was extracted with DCM (3 × 40 mL) and washed with brine (3 × 20 mL). The organic phase was collected, dried over MgSO₄, filtered and concentrated under reduced pressure. Purification by flash chromatography (DCM/MeOH 98/2) afforded 0.300 g (26%) of a red solid. R*_{f}* = 0.45 (DCM/MeOH 98/2); ¹H NMR (600 MHz, CDCl₃) δ 8.63 (d, *J* = 8.0 Hz, 4H), 7.50 (d, *J* = 7.0 Hz, 4H), 5.11 - 4.38 (m, 8H), 3.97 - 3.26 (m, 4H), 1.95-0.57 (m, 18H); ¹³C NMR (151 MHz, CDCl₃) δ 163.77, 155.61, 155.57, 143.70, 130.81, 128.51, 128.21, 127.87, 83.18 (d, *J* = 167.9 Hz), 82.61 (d, *J* = 169.3 Hz), 72.44, 65.78, 52.09, 51.09, 47.67 (d, *J* = 19.9 Hz), 47.19 (d, *J* = 21.0 Hz), 28.38.

### N,N'-(((1,2,4,5-Tetrazine-3,6-diyl)bis(4,1-phenylene))bis(methylene))bis(2-fluoroethan-1-amine) (Tz-I)

Di-*tert*-butyl (((1,2,4,5-tetrazine-3,6-diyl)bis(4,1-phenylene))bis(methylene))bis((2-fluoroethyl) carbamate) (0.15 gr, 0.25 mmol) was treated with a solution of HCl (4 M) in dioxane (1 mL). A precipitate was formed. Filtration afforded 0.1 gr (83%) of the desired product as hydrochloride salt. ¹H NMR (600 MHz, DMSO) δ 9.70 (s, 4H), 8.61 (d, *J* = 8.0 Hz, 4H), 7.89 (d, *J* = 8.0 Hz, 4H), 4.86 (t, *J* = 4.6 Hz, 2H), 4.78 (t, *J* = 4.6 Hz, 2H), 4.37 (s, 4H), 3.40 - 3.25 (m, 4H); ¹³C NMR (151 MHz, DMSO) δ 163.63, 136.83, 132.81, 131.66, 128.28, 80.09 (d, *J* = 165.1 Hz), 50.24, 47.21 (d, *J* = 19.8 Hz).

### Example 1.2

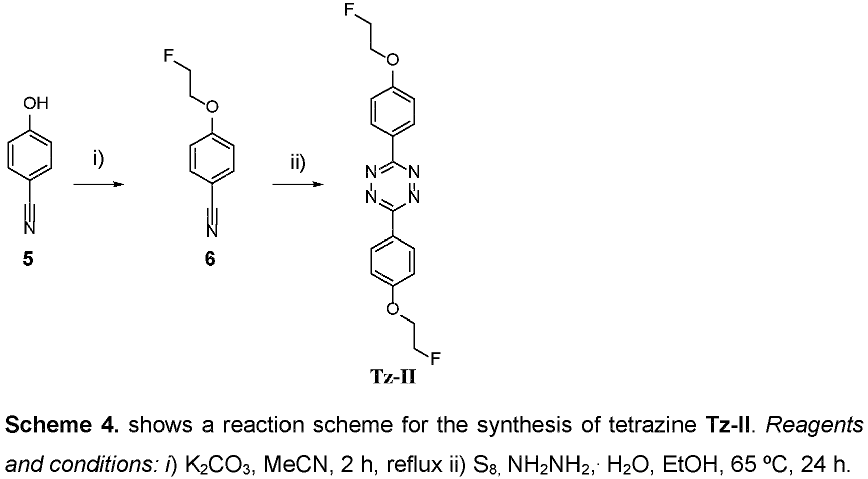

### Synthesis of 4-(2-fluoroethoxy)benzonitrile (6)

To a solution of 4-hydroxybenzonitrile (0.6 g, 5.00 mmol) and K₂CO₃ (1.38 g, 10.00 mmol) in CH₃CN (20 mL) was added 1-fluoro-2-iodoethane (1.04 g, 6.00 mmol). The reaction was refluxed for 12 h and then concentrated under reduced pressure. The resulting mixture was diluted with water (50 mL), extracted with EtOAc (3 × 50 mL), washed with brine (50 mL), dried over MgSO₄, filtered and concentrated under reduced pressure. Purification by flash chromatography (Heptane/EtOAc 80/20) afforded 0.8 g (97%) of the desired compound as a yellow oil. Rf = 0.37 (Heptane/EtOAc 70/30); ¹H NMR (400 MHz, CDCl₃) δ 7.62 (d, *J* = 8.9 Hz, 1H), 7.01 (d, *J* = 8.9 Hz, 1H), 5.05 - 4.79 (m, 1H), 4.77 - 4.71 (m, 1H), 4.36 - 4.29 (m, 1H), 4.29 - 4.22 (m, 1H); ¹³C NMR (101 MHz, CDCl₃) δ 161.69, 134.06, 119.03, 115.33, 104.59, 81.48 (d, *J* = 171.7 Hz), 67.37 (d, *J* = 20.5 Hz).

### Synthesis of 3,6-bis(4-(2-fluoroethoxy)phenyl)-1,2,4,5-tetrazine (Tz-II)

To a suspension of 4-(2-fluoroethoxy)benzonitrile (0.65 gr, 3.95 mmol) and Zn(OTf)₂ (0.72 gr, 1.98 mmol) in EtOH (30 mL) was added hydrazine monohydrate (3.83 mL, 79 mmol). The mixture was allowed to stir at 70 °C for 22 hours, and when the reaction is completed, is cooled at room temperature. The volatiles were removed under reduced pressure and the residue solubilized in EtOH (40 mL). A solution of NaNO₂ (5.52 g, 80.00 mmol ) in water (20 mL) was added to the crude reaction followed by dropwise addition of HCl (2M) until gas evolution ceased and a pH of 2-3 was achieved producing a red mixture. The crude reaction was extracted with DCM (3 × 40 mL) and washed with brine (3 × 20 mL). The organic phase was collected, dried over MgSO₄, filtered and concentrated under reduced pressure. Purification by flash chromatography (DCM/MeOH 98/2) afforded 0.23 g (33%) of a red solid. R*_{f}* = 0.38 (DCM/MeOH 99/1); ¹H NMR (400 MHz, DMSO) δ 8.49 (d, *J* = 9.0 Hz, 2H), 7.28 (d, *J* = 9.0 Hz, 2H), 5.17 - 4.68 (m, 1H), 4.62 - 4.28 (m, 1H)

### 3-(5-Iodopyridin-2-yl)-6-(pyridin-2-yl)-1,2,4,5-tetrazine (Tz-VII)

The compound was obtained following the procedure reported in Albu et al. 125I-Tetrazines and Inverse-Electron-Demand Diels-Alder Chemistry: A Convenient Radioiodination Strategy for Biomolecule Labeling, Screening, and Biodistribution Studies. Bioconjug Chem. 2016, ;27, 207-216.

### 2,2'-((2-(2-Fluoroethoxy)-4-(1,2,4,5-tetrazin-3-yl)benzyl)azanediyl)diacetic acid (Tz-XI)

The compound was obtained following the procedure reported in Battisti et al. Development of the First Aliphatic 18F-Labeled Tetrazine Suitable for Pretargeted PET Imaging-Expanding the Bioorthogonal Tool Box. J. Med. Chem. 2021, 64, 20, 15297-15312.

### Example 1.3

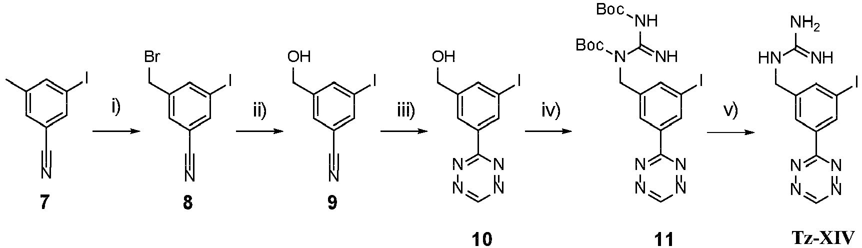

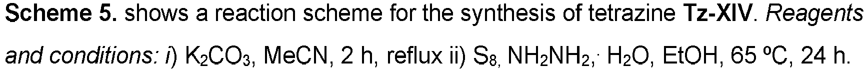

### 3-(Bromomethyl)-5-iodobenzonitrile (8)

In a 100mL round-bottomed flask fitted with a stir bar, 3-iodo-5-methylbenzonitrile (2 g, 8.23 mmol), N-bromosuccinmide (2.20 g, 12.34 mmol) and AIBN (540 mg, 3.29 mmol) were dissolved in MecN (25 mL). The reaction was refluxed at 80-85°C for 6h. Water and DCM were added. The organic layer was washed twice with water, and each water phase was extracted twice with DCM. The combined organic phase was washed in brine, dried over Mg₂SO₄, filtered and concentrated under vacuum. The residue was dissolved in DCM and absorbed unto Celite. Next, the crude product was purified by flash chromatography (heptane/DCM 85:15 -> 70:30) to give 2g (75.6%) of NB35 as a white solid. R*_{f}* = 0.11(heptane:DCM 8:2). ¹H-NMR (CDCl₃, 600MHz): 4.38 (s, 2H), 7.65 (t, 1H, *J* = 1.6), 7.91 (t, 1H, *J* = 1.55), 7.97 (t, 1H, *J* = 1.65). ¹³C-NMR (CDCl₃, 600 MHz): 30.0, 94.2, 114.7, 116.7, 131.8, 140.29, 141.1, 142.4.

### 3-(Hydroxymethyl)-5-iodobenzonitrile (9)

3-(Bromomethyl)-5-iodobenzonitrile (1 g, 3.11 mmol), was refluxed (75-80 °C) in 1:1 MeCN/deionized water (24 mL) with barium carbonate (1.23 g, 6.21 mmol). The reaction was stirred overnight. LCMS showed reaction progression, more MeCN. After 28h the reaction was still slowly progressing, more MeCN, water and barium carbonate (1 eq) were added. The reaction was left stirring with reflux overnight. After 45h, complete reaction was observed by LCMS. The mixture was cooled to room temperature. The solids were removed by filtration. The filtrate was extracted three times with DCM. The combined organic phases were dried for Mg₂SO₄, filtered and concentrated. The residue was re-dissolved in DCM and absorbed unto Celite. The compound was purified by flash chromatography (5-20% EtOAc in heptane) to afford a white solid. Yield: 582 mg (69%). R*_{f}* = 0.36 (7:3 heptane:EtOAc). ¹H-NMR (CDCl₃, 600MHz): 1.88 (t, 1H, *J* = 6.76), 4.72 (d, 2H, *J* = 5.7), 7.62 - 7.64 (m, 1H), 7.88 - 7.90 (m, 1H), 7-95 -7.97 (m, 1H). ¹³C-NMR (CDCl₃, 600 MHz): 63.3, 94.1, 114.4, 117.2, 129.4, 139.47, 140.1, 144.2.

### (3-Iodo-5-(1,2,4,5-tetrazin-3-yl)phenyl)methanol (10)

3-(hydroxymethyl)-5-iodobenzonitrile (548 mg, 2.12 mmol), DCM (135 uL, 2.12 mmol), S₈ (136 mg, 0.53 mmol) and ethanol (3 mL) were mixed together in a 20 mL microwave reaction tube. Hydrazine monohydrate (825 uL, 16.9 mmol) was added slowly with stirring afterwards. The vessel was sealed and the reaction mixture was heated to 50 °C for 22 hours. The reaction turned dark with yellow precipitate. Afterwards 5 mL of DCM and sodium nitrite (1.46 g, 21.15 mmol) in 20 mL of H₂O were added to the mixture. Excess acetic acid (6 mL) was then added slowly during which the solution turned bright red in color. The reaction mixture was left stirring for about 10 min. Then, the water phase was extracted 4 times with DCM. The organic phase was dried over anhydrous magnesium sulfate, filtered, concentrated under reduced pressure. The resulting residue (769 mg) was purified using flash chromatography (heptane/EtOAc) to yield 262mg (39%) of product as a red/pink solid. R*_{f}* = 0.24 (Heptane/EtOAc 70/30). ¹H-NMR(CDCl₃, 600MHz): 1.88 (t, 1H, *J* = 5.9), 4.82 (d, 2H, *J* = 4.8Hz), 8.03 - 8.05 (m, 1H), 8.59 - 8.60 (m, 1H), 8.90 - 8.93 (m, 1H), 10.26 (s, 1H). ¹³C-NMR (CDCl₃, 600 MHz): 64.1, 95.2, 125.8, 133.7,136.3, 140.3, 144.4, 158.2, 165.4.

### Di-tert-butyl carbamimidoyl(3-iodo-5-(1,2,4,5-tetrazin-3-yl)benzyl)carbamate (11)

(3-iodo-5-(1,2,4,5-tetrazin-3-yl)phenyl)methanol (184mg, 0.59mmol) in 8 mL anhydrous THF was stirred under argon with 1,3-bis(*tert*-butoxycarboxyl)guanidine (167 mg, 0.64 mmol) and triphenylphosphine (310 mg, 1.17 mmol). DIAD (230 uL, 1.17 mmol) was added dropwise. First, the mixture turned dark, but after 15min, it was red/pink from the Tz. After 3h, LCMS showed complete conversion. The solvent was evaporated, and the resulting crude was reconstituted in EtOAc, washed with 2x water and 2x brine, dried over Mg₂SO₄, filtered and concentrated. The compound was dissolved in EtOAc and absorbed unto Celite. The compound was purified by flash chromatography (95:5 Heptane: EtOAc). Yield 186mg(45%). RF= 0.36 (8:2 Heptane:EtOAc). ¹H-NMR (CDCl₃, 400MHz): 1.45 (s, 9H), 1.52 (s, 9H), 5.23 (s, 2H), 8.05 - 8.08 (m, 1H), 8.60 - 8.63 (m, 1H), 8.89 (s, 1H), 9.25 (s, 1H), 9.43 (s, 1H), 10.24 (s, 1H). ¹³C-NMR (CDCl₃, 400 MHz): 28.1, 28.5, 46.9, 79.3, 85.0, 94.8, 127.2, 133.4, 135.9, 142.0, 142.2, 154.7, 158.2, 160.5, 163.7, 165.4.

### 1-(3-Iodo-5-(1,2,4,5-tetrazin-3-yl)benzyl)guanidine (Tz-XIV)

di-*tert*-butyl carbamimidoyl(3-iodo-5-(1,2,4,5-tetrazin-3-yl)benzyl)carbamate (30 mg, 0.054 mmol) was deprotected using 4 M HCl in dioxane (4 mL) for 41h, after which the residue was concentrated under vacuum. The crude residue was dissolved in 5 mL H₂O containing 0.1% TFA and was submitted to prep-HPLC. Fractions containing the pure product were combined and freeze-dried. Yield 17 mg (1xTFA: 69%, 2xTFA: 56%). ¹H-NMR (MeOD, 600MHz): 4.55 (s, 2H), 8.02 - 8.03 (m, 1H), 8.56 - 8.57 (m, 1H), 8.88 - 8.90 (m, 1H), 10.40 (s, 1H). ¹³C-NMR (MeOD, 600 MHz): 44.9, 95.8, 126.8, 136.0, 137.3, 141.3, 141.5, 158.9, 159.7, 166.4.

### 3-(4-Iodophenyl)-1,2,4,5-tetrazine (Tz-XVI)

The compound was obtained following the procedure reported in WO2020/108720 - Novel tetrazine compounds for in vivo imaging.

### 3-(4-(2-Fluoroethoxy)phenyl)-1,2,4,5-tetrazine (Tz-XII)

The compound was obtained following the procedure reported in García-Vázquez et al. Development of 18F-Labeled Bispyridyl Tetrazines for In Vivo Pretargeted PET Imaging, Pharmaceuticals 2022, 15 (2), 245.

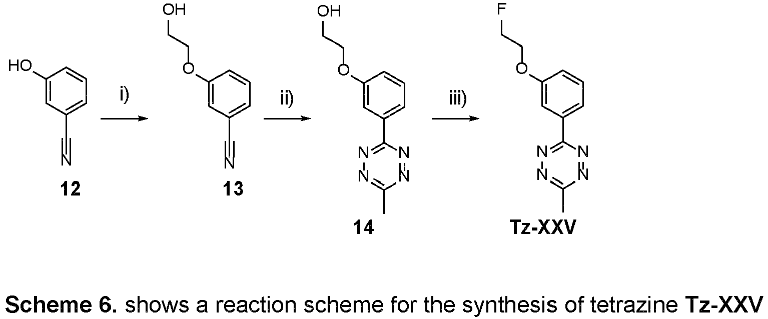

### 3-(2-hydroxyethoxy)benzonitrile (13)

To a solution of 3-hydroxybenzonitrile (2.67 g, 22.41 mmol) and K₂CO₃ (9.29 g, 67.24 mmol) in CH₃CN (100 mL) was added 2-bromorthanol (8.41 g, 67.24 mmol). The reaction was refluxed for 12 h and then concentrated under reduced pressure. The resulting mixture was diluted with water (150 mL), extracted with EtOAc (3 × 150 mL), washed with brine (50 mL), dried over MgSO₄, filtered and concentrated under reduced pressure. Purification by flash chromatography (Heptane/EtOAc 60/40) afforded 3.5 g (96%) of the desired compound as a white solid. Rf = 0.25 (Heptane/EtOAc 50/50); ¹H NMR (400 MHz, CDCl₃) δ 7.48 - 7.34 (m, 1H), 7.31 - 7.24 (m, 1H), 7.18 - 7.13 (m, 2H), 4.10 (dd, *J* = 5.2, 3.8 Hz, 2H), 3.99 (dd, *J* = 5.2, 3.8 Hz, 2H), 2.20 (s, 1H); ¹³C NMR (101 MHz, CDCl₃) δ 158.77, 130.46, 124.93, 119.79, 117.56, 113.32, 69.63, 61.18.

### 2-(3-(6-methyl-1,2,4,5-tetrazin-3-yl)phenoxy)ethan-1-ol (14)

Acetonitrile (1.76 mL, 33.70 mmol), Zn(OTf)₂ (1.22 g, 3.37 mmol), hydrazine monohydrate (6.54 mL, 6.79 mmol) and the 3-(2-hydroxyethoxy)benzonitrile (1.1 g, 6.74 mmol) were added in a microwave vial and sealed. The mixture was allowed to stir at 60°C for 22 hours, and when the reaction is completed, is cooled at room temperature and unsealed. A solution of NaNO₂ (9.31 g, 134.82 mmol) in water (100 mL) was added to the crude reaction followed by dropwise addition of HCl (2M) until gas evolution ceased and a pH of 3-2 was achieved producing a red mixture. The crude reaction was extracted with EtOAc (3 × 50 mL) and washed once with brine (100 mL). The organic phase was collected, dried with MgSO₄, filtered and concentrated *in vacuo.* The tetrazine was purified via flash chromatography using n-Heptane:EtOAC (40/20) as eluent to give 0.48 g (31%) of the compound as a red solid. Rf = 0.28 (50/50 heptane/EtAOc), ¹H NMR (600 MHz, DMSO) δ 8.06 (dt, *J* = 7.7, 0.9 Hz, 1H), 7.98 (dd, *J* = 2.7, 1.6 Hz, 1H), 7.58 (t, *J* = 8.0 Hz, 1H), 7.28 (ddd, *J* = 8.3, 2.7, 0.9 Hz, 1H), 4.92 (t, *J* = 5.5 Hz, 1H), 4.13 (t, *J* = 4.9 Hz, 2H), 3.81 - 3.75 (m, 2H); ¹³C NMR (151 MHz, DMSO) δ 167.70, 163.59, 159.88, 133.67, 131.18, 120.21, 119.39, 113.22, 70.36, 60.00, 21.31.

### 3-(3-(2-fluoroethoxy)phenyl)-6-methyl-1,2,4,5-tetrazine (Tz-XXV)

To a solution of 2-(3-(6-methyl-1,2,4,5-tetrazin-3-yl)phenoxy)ethan-1-ol (0.15 g, 0.69 mmol) in dry THF (10 mL) was added DAST (0.19 mL, 1.37 mmol) at -40 °C. The reaction was stirred at rt for 4 h. The solution was diluted with saturated NaHCOs and extracted with DCM (3 × 10 mL). Purification by flash chromatography (80/20 heptane/EtOAc) afforded 0.07 g (46%) of the desired compound as a red solid. Rf = 0.32 (80/20 Heptane/EtOAc); ¹H NMR (600 MHz, CDCl₃) δ 10.22 (s, 1H), 8.27 (dt, *J* = 7.8, 1.3 Hz, 1H), 8.18 (dd, *J* = 2.7, 1.6 Hz, 1H), 7.53 (t, *J* = 8.0 Hz, 1H), 7.24 (ddd, *J* = 8.3, 2.7, 1.0 Hz, 1H), 4.88 - 4.83 (m, 1H), 4.80 - 4.75 (m, 1H), 4.40 - 4.37 (m, 1H), 4.37 - 4.32 (m, 1H); ¹³C NMR (151 MHz, CDCl₃) δ 166.25, 159.26, 157.90, 132.92, 130.65, 121.46, 120.67, 112.97, 81.78 (d, *J=* 171.1 Hz), 67.43 (d, *J* = 20.4 Hz).

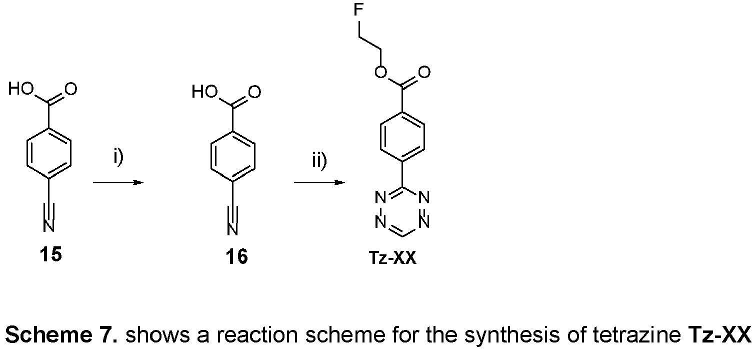

### Synthesis of XXX

### 4-(1,2,4,5-tetrazin-3-yl)benzoic acid (16)

4-cyanobenzoic acid (0.3 g, 2.00 mmol), CH₂Cl₂ (0.12 mL, 2.00 mmol), sulfur (0.12 g, 0.5 mmol) and ethanol (4.0 mL) were mixed together in a microwave reaction vial. Hydrazine monohydrate (0.82 mL, 16.00 mmol) was added dropwise with stirring. The vessel was sealed and the reaction mixture was heated to 50 °C for 24 hours. The reaction was diluted with 3 ml of CH₂Cl₂ and sodium nitrite (1.44 g, 20.00 mmol) in 20 ml of H₂O was added dropwise to the mixture under cooling. Excess acetic acid (7 mL) was then added slowly during which the solution turned bright red in color. The reaction mixture was extracted with CH₂CL₂ (3 × 20 mL). The organic phase was dried over MgSO₄ and concentrated under reduced pressure. The resulting residue was purified using flash chromatography (CH₂Cl₂/MeOH 98/2) to give 0.08 g (20%) of the desired product as a pink solid. Rf = 0.31 (CH₂Cl₂/MeOH 95/5); ¹H NMR (400 MHz, DMSO) δ 13.32 (s, 1H), 10.66 (s, 1H), 8.62 (d, *J* = 8.5 Hz, 2H), 8.22 (d, *J* = 8.5 Hz, 2H); ¹³C NMR (101 MHz, DMSO) δ 166.67, 165.08, 158.24, 135.70, 134.32, 130.20, 127.97.

### 2-Fluoroethyl 4-(1,2,4,5-tetrazin-3-yl)benzoate (Tz-XX)

4-(1,2,4,5-Tetrazin-3-yl)benzoic acid (0.04 g, 0.20 mmol) and 1-fluoro-2-iodoethane (0.05 g, 0.30 mmol) was dissolved in 2 ml dry DMF and DIPEA (0.10 mL, 0.60 mmol) was added. The reaction was left at 70 °C overnight. The reaction was diluted with CH₂Cl₂ (15 mL) and washed with NH₄Cl and H₂O (2x10 mL). The organic phase was dried over MgSO₄ and concentrated under reduced pressure. Purification by flash chromatography (85/15 Heptane/EtOAc) afforded 0.04 g (81%) of **3** as a red solid. Rf = 0.23 (Heptane/EtOAc 80/20); ¹H NMR (600 MHz, CDCl₃) δ 10.21 (s, 1H), 8.65 (d, *J* = 8.6 Hz, 2H), 8.23 (d, *J* = 8.5 Hz, 2H), 4.89 - 4.72 (m, 1H), 4.69 - 4.64 (m, 1H), 4.63 - 4.57 (m, 1H), 4.57 - 4.50 (m, 1H); ¹³C NMR (101 MHz, CDCl₃) δ 165.92, 165.50, 157.99, 135.75, 133.63, 130.58, 128.27, 81.26 (d, *J* = 171.1 Hz), 64.30 (d, *J* = 20.1 Hz).

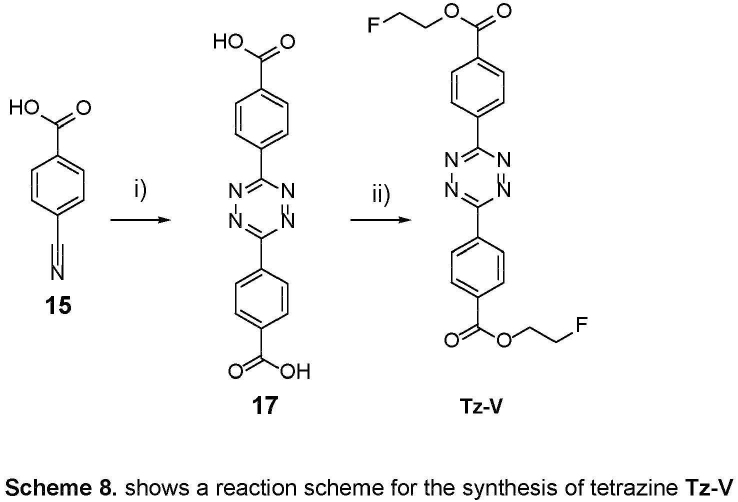

### 4,4'-(1,2,4,5-Tetrazine-3,6-diyl)dibenzoic acid (17)

4-Cyanobenzoic acid (1.00 g, 6.80 mmol, 1.00 equiv.) and Zn(OTf)₂ (494 mg, 1.36 mmol, 0.20 equiv.) were suspended in hydrazine monohydrate (3.30 mL, 67.97 mmol, 10.00 equiv.). The mixture was allowed to stir at 60 °C for 22 hours, and when the reaction is completed, is cooled at room temperature. The reaction mixture was diluted with DCM (20 mL) and a solution of NaNO₂ in water (20 mL) was added to the crude reaction followed by dropwise addition of acetic acid until gas evolution ceased and a pH of 3-2 was achieved producing a red mixture. The crude reaction was filtered and the residue was washed with 25 mL water, 50 mL EtOAc, 50 mL DCM and 50 mL MeOH. The residue was dried under vacuum and the crude was used in the next reaction, without purification, which yielded the product as a pink solid (1.36 g, 4.21 mmol, 62%). ¹H NMR (600 MHz, DMSO) δ 8.55 (d, *J* = 8.2 Hz, 2H), 8.16 (d, *J* = 8.2 Hz, 2H), 7.94 (d, *J* = 8.4 Hz, 2H), 7.86 (d, *J* = 8.2 Hz, 2H).

### Bis(2-fluoroethyl) 4,4'-(1,2,4,5-tetrazine-3,6-diyl)dibenzoate (Tz-V)

4,4'-(1,2,4,5-Tetrazine-3,6-diyl)dibenzoic acid (100 mg, 0.31 mmol, 1.00 equiv.) and 2-fluoro2-iodoethane (101 µL, 1.24 mmol, 4 equiv.) were dissolved in dry DMF (15 mL) and DIPEA (322 µL, 1.86 mmol, 6 equiv.) was added dropwise and the reaction was stirred at 80 °C, 5 days. After completion, the reaction mixture was cooled down, diluted with water (100 mL) and extracted with DCM (2 × 50 mL). The crude was purified by flash chromatography, which yielded the product as a pink solid (73 mg, 0.18 mmol, 57%). ¹H NMR (600 MHz, DMSO) δ 8.73 (d, *J* = 8.8 Hz, 4H), 8.30 (d, *J* = 8.8 Hz, 4H), 4.87 - 4.83 (m, 2H), 4.80 - 4.75 (m, 2H), 4.66 - 4.62 (m, 2H), 4.60 - 4.57. (m, 2H); ¹³C NMR (151 MHz, DMSO) δ 165.0, 162.9, 136.1, 132.7, 130.3, 128.1, 82.2, 81.1, 64.5, 64.4.

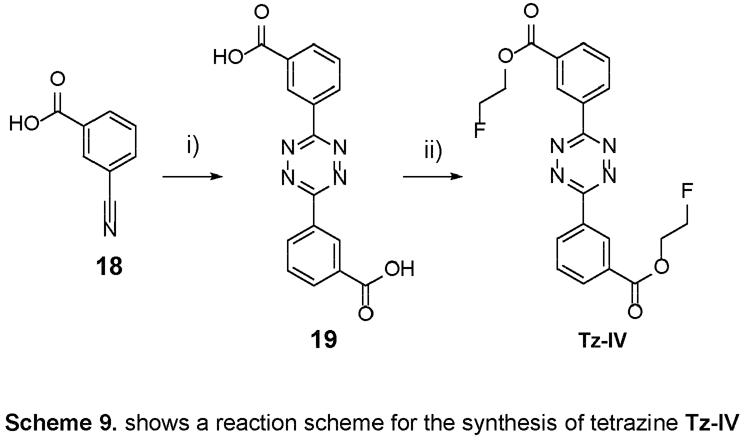

### 3,3'-(1,2,4,5-Tetrazine-3,6-diyl)dibenzoic acid (19)

3-Cyanobenzoic acid (500 mg, 3.40 mmol, 1.00 equiv.) and Zn(OTf)₂ (247 mg, 0.69 mmol, 0.0 equiv.) were suspended in hydrazine monohydrate (1.65 mL, 33.98 mmol, 10.00 equiv.). The mixture was allowed to stir at 60 °C for 22 hours, and when the reaction is completed, is cooled at room temperature. The reaction mixture was diluted with DCM (20 mL) and a solution of NaNO₂ in water (20 mL) was added to the crude reaction followed by dropwise addition of acetic acid until gas evolution ceased and a pH of 3-2 was achieved producing a red mixture. The crude reaction was filtered and the residue was washed with 25 mL water, 50 mL EtOAc, 50 mL DCM and 50 mL MeOH. The residue was dried under vacuum and the crude was used in the next reaction, without purification, which yielded the product as a pink solid (846 mg, 2.63 mmol, 77%). ¹H NMR (600 MHz, DMSO) δ 9.09 (t, *J* = 1.8 Hz, 2H), 8.75 (dt, *J* = 7.8, 1.5 Hz, 2H), 8.26 (dt, *J* = 7.7, 1.5 Hz, 2H), 7.82 (t, *J* = 7.7 Hz, 2H); ¹³C NMR (151 MHz, DMSO) δ 166.7, 163.1, 133.1, 132.2, 131.3, 129.9, 128.3.

### Bis(2-fluoroethyl) 3,3'-(1,2,4,5-tetrazine-3,6-diyl)dibenzoate (Tz-IV)

3,3'-(1,2,4,5-Tetrazine-3,6-diyl)dibenzoic acid (100 mg, 0.31 mmol, 1.00 equiv.) and 2-fluoro2-iodoethane (101 µL, 1.24 mmol, 4.00 equiv.) were dissolved in dry DMF (5 mL) and DIPEA (322 µL, 1.86 mmol, 6.00 equiv.) was added dropwise to the reaction was stirred at 80 °C, 5 days. The reaction mixture was cooled down to RT, diluted with water (50 mL) and extracted with DCM (2 × 50 mL). The crude was purified by flash chromatography, which yielded the product as a pink solid (89 mg, 0.21 mmol, 69%). ¹H NMR (600 MHz, CDCl₃) δ 9.38 (d, *J* = 1.8 Hz, 2H), 8.89 (dt, *J* = 7.9, 1.6 Hz, 2H), 8.38 (dt, *J* = 7.8, 1.5 Hz, 2H), 7.75 (t, *J* = 7.8 Hz, 2H), 4.85 - 4.82 (m, 2H), 4.77 - 4.74 (m, 2H), 4.69 - 4.67 (m, 2H), 4.65 - 4.62 (m, 2H). ¹³C NMR (151 MHz, CDCl₃) δ 165.69, 163.80, 134.05, 132.55, 132.33, 131.28, 129.82, 129.53, 81.98, 80.84, 64.51, 64.38.

### Example 2

### Synthesis of cyclic unsaturated hydrocarbon with at least one double bond in the cis-configuration and their precursors

### Example 2.1

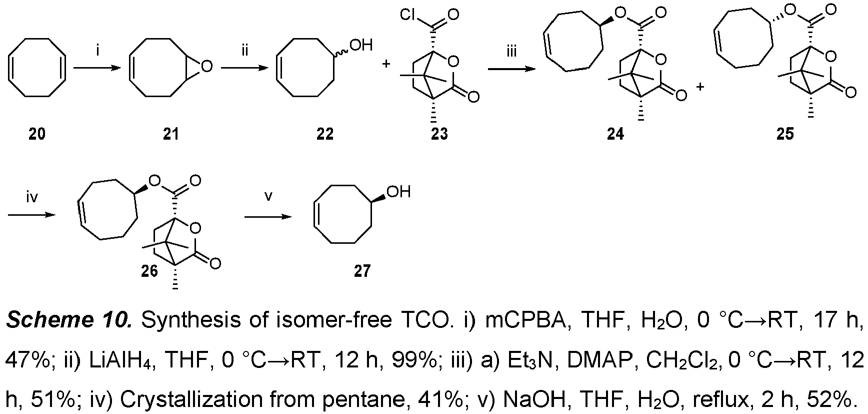

### (Z)-9-Oxabicyclo[6.1.0]non-4-ene (21)

Cis,cis-1,5-cyclooctadiene (22.0 g, 203.36 mmol, 1.00 equiv.) and dry CH₂Cl₂ (300 mL) were added to a 500 mL round-bottom flask. The mixture was cooled to 0 °C with an ice bath and mCPBA (45.57 g, 203.36 mmol, 1.00 equiv.) was added portion wise to give a white suspension. The mixture was allowed to reach room temperature and left stirring overnight. The mixture was filtered and washed with NaHCOs saturated solution (3 × 100 mL) and NaCl saturated solution (1 × 100 mL). The organic layer was collected, dried with MgSO₄, filtered and concentrated under reduced pressure. Purification by flash chromatography (n-heptane/EtOAc, 90:10) yielded (*Z*)-9-oxabicyclo[6.1.0]non-4-ene (11.82 g, 95.16 mmol, 47%) as a colorless oil. ¹H NMR (600 MHz, CDCl₃) δ 5.69 - 5.48 (m, 2H), 3.15 - 2.91 (m, 2H), 2.55 - 2.35 (m, 2H), 2.21 -2.08 (m, 2H), 2.08-1.86 (m, 4H); ¹³C NMR (151 MHz, CDCl₃) δ 129.00, 56.87, 28.25, 23.82.

### (Z)-Cyclooct-4-enol (22)

Lithium aluminum hydride tablets (3.26 g, 85.93 mmol, 3.00 equiv.) were added to an oven-dried 500 mL three-necked round-bottom flask. The flask was sealed and flushed with argon. The flask was cooled to 0 °C using an ice-bath and dry THF (120 mL) was added slowly while vigorously stirring to give a grey suspension. 1,2-Epoxy-5-cyclooctene (3.56 g, 28.64 mmol, 1.00 equiv.) in dry THF (10 mL) was added dropwise and the mixture was allowed to reach room temperature and stirred overnight. The mixture was cooled to 0 °C in an ice bath and quenched with EtOAc (120 mL). A saturated solution of Rochelle salt (100 mL) was added, and the mixture was stirred vigorously for 10 minutes. The mixture was transferred to a separatory funnel and the organic layer was collected. The aqueous layer was extracted with DCM (3 × 150 mL). The combined organic layers were washed with H₂O (200 mL), dried over MgSO₄, filtered and concentrated under reduced pressure to give (Z)-Cyclooct-4-enol (3.49 g, 28.45 mmol, 99%). ¹H NMR (600 MHz, CDCl₃) δ 5.75 - 5.63 (m, 1H), 5.61 - 5.52 (m, 1H), 3.86 - 3.75 (m, 1H), 2.36 - 2.24 (m, 1H), 2.20 - 2.04 (m, 3H), 1.97 (s, 1H), 1.93 - 1.88 (m, 1H), 1.86 - 1.81 (m, 1H), 1.75 - 1.68 (m, 1H), 1.67 - 1.59 (m, 1H), 1.56 - 1.46 (m, 2H); ¹³C NMR (151 MHz, CDCl₃) δ 130.23, 129.63, 72.85, 37.75, 36.36, 25.75, 24.97, 22.88.

### (Z)-cyclooct-4-en-1-yl (1R,4S)-4,7,7-trimethyl-3-oxo-2-oxabicyclo[2.2.1]heptane-1-carboxylate (Epimers mixture) (24)

(±)-(*Z*)-Cyclooct-4-enol (4.5 g, 35.65 mmol) was dissolved in dry CH₂Cl₂ (100 mL), to which was added DMAP (0.87 g, 7.13 mmol) and Et₃N (14.9 mL, 106.97 mmol). The solution was cooled to 0°C and (1S)-(-)-camphanic chloride (8.5 g, 38.22 mmol) was added portion wise to the mixture. The resulting solution was allowed to stir at room temperature for 17 hours. The mixture was washed with NaHCO₃ saturated solution (3 × 100 mL) and NaCl saturated solution (1 × 100 mL). The organic layer was collected, dried with MgSO₄, filtered and concentrated under reduced pressure to give 5.63 g (51%) of a mixture of the desired epimers. Recrystallization from pentane afforded 2.31 g (41%) of (*Z*)-cyclooct-4-en-1-yl (1*R*,4*S*)-4,7,7-trimethyl-3-oxo-2-oxabicyclo[2.2.1]heptane-1-carboxylate as crystals (needles). ¹H NMR (400 MHz, CDCl₃) δ 5.75 - 5.58 (m, 2H), 5.05 - 4.94 (m, 1H), 2.46 - 2.29 (m, 2H), 2.28 - 2.08 (m, 3H), 2.06 - 1.84 (m, 4H), 1.84 - 1.74 (m, 1H), 1.74 - 1.33 (m, 4H), 1.10 (s, 3H), 1.04 (s, 3H,). 0.95 (s, 3H); ¹³C NMR (101 MHz, CDCl₃) δ 178.46, 166.97, 130.05, 129.99, 129.59, 129.56, 91.23, 54.96, 54.21, 33.87, 33.85, 33.81, 30.69, 29.12, 29.10, 25.67, 25.65, 24.92, 24.88, 22.37, 22.35, 17.01, 16.95, 16.88, 9.84.

### (S,Z)-cyclooct-4-en-1-ol (27)

(*S,Z*)-cyclooct-4-en-1-yl (1*R*,4*S*)-4,7,7-trimethyl-3-oxo-2-oxabicyclo[2.2.1]heptane-1-carboxylate (0.26 g, 0.85 mmol) was dissolved in THF (10 mL), to which was added a solution of NaOH (0.2 g, 4.24 mmol) in H₂O (1 mL). The reaction was vigorously stirred at reflux for 2 hours. The mixture was quenched with H₂O (10 mL) and the aqueous layer was extracted with DCM (3 × 30 mL). The combined organic layers were washed with H₂O (20 mL), dried over MgSO₄, filtered and concentrated under reduced pressure to give (S,Z)-Cyclooct4enol (0.056 g, 52%) as a colorless oil. ¹H NMR (600 MHz, CDCl₃) δ 5.75 - 5.63 (m, 1H), 5.61 - 5.52 (m, 1H), 3.86 - 3.75 (m, 1H), 2.36 - 2.24 (m, 1H), 2.20 - 2.04 (m, 3H), 1.97 (s, 1H), 1.93 - 1.88 (m, 1H), 1.86 - 1.81 (m, 1H), 1.75 - 1.68 (m, 1H), 1.67 - 1.59 (m, 1H), 1.56 - 1.46 (m, 2H); ¹³C NMR (151 MHz, CDCl₃) δ 130.23, 129.63, 72.85, 37.75, 36.36, 25.75, 24.97, 22.88.

### Example 2.2

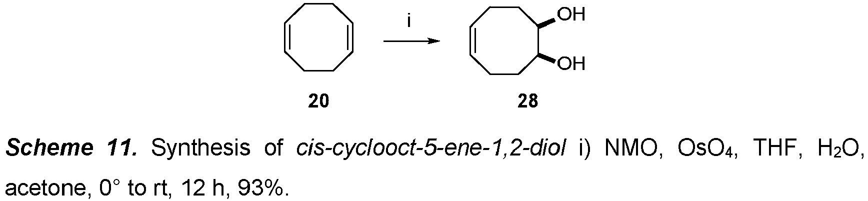

### Cis-Z-cyclooct-5-ene-1,2-diol (28)

To a stirred mixture of 1,5-cyclooctadiene (1 1.22 mL, 12.9 mmol), 4-methylmorpholine *N*-oxide (1.87 g, 13.75 mmol) and THF: H₂O : acetone (1:1:1) (90 mL) at 0 °C was added osmium tetroxide (12.7 mg, 0.05 mmol). After 12 h at 25 °C the reaction mixture was poured into an aqueous saturated solution of NaHSOs (60 mL), extracted with EtOAc (3 × 150 mL), washed with water (2 × 50 mL) and brine (50 mL). Drying (MgSO₄) and concentration followed by flash chromatography (silica, 30% EtOAc in heptane) afforded the desired compound 1.70 g (93%).¹H NMR (400 MHz, CDCl₃) δ 5.72 - 5.61 (m, 2H), 4.03 - 3.96 (m, 2H), 2.56 - 2.44 (m, 2H), 2.10 - 1.96 (m, 4H), 1.86-1.74 (m, 2H); ¹³C NMR (101 MHz, CDCl₃) δ 130.09, 75.18, 32.08, 23.11.

### Example 3

### Synthesis of C₇ - C₉ cyclenes with at least one double bond in the cis-configuration and their precursors

### Synthesis of unsaturated heterocycle, with at least one double bond in the trans-configuration and their precursors

### Example 3.1

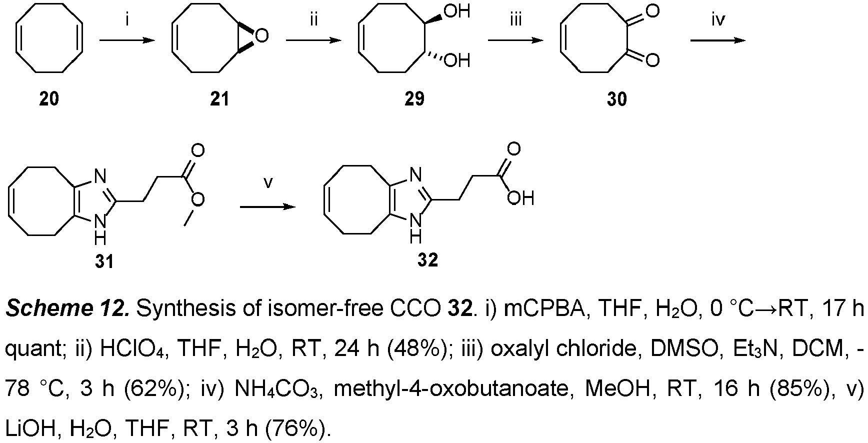

**(*Z*)-Cyclooct-5-ene-1,2-diol (29).** 25 (1.15 g, 9.24 mmol) was dissolved in THF/water (3:2 v/v, 50 mL) mixture containing HClO₄ (250 µL, 2.90 mmol). The reaction was stirred at room temperature for 24 h. Water (10 mL) was added to the reaction mixture, and the product was extracted with 3 × Et₂O (20 mL). The combined ether fractions were washed with 2 × 2M NaHCOs (25 mL). The crude was purified by CombiFlash, which yielded a clear viscous oil. (624 mg, 4.40 mmol, 48%). ¹H NMR (600 MHz, Chloroform-d) δ 5.64 - 5.56 (m, 2H), 3.72 - 3.65 (m, 2H), 2.41 - 2.33 (m, 2H), 2.17 - 2.09 (m, 4H), 1.63 - 1.55 (m, 2H); ¹³C NMR (151 MHz, Chloroform-*d*) δ 129.3, 74.1, 33.6, 22.9.

### (Z)-Cyclooct-5-ene-1,2-dione (30)

Under Argon atmosphere, at -78 °C, a solution of DMSO (1.05 mL, 14.77 mmol) in dry DCM (30 mL) was added drop wise to a solution of (COCl)₂ (736 µL, 8.44 mmol) in dry DCM (15 mL). After addition, the reaction mixture is stirred for 30 min at -78 °C. A solution of **29** (500 mg, 3.52 mmol) in dry DCM (10 mL) is added drop wise and the reaction mixture is stirred an additional 30 minutes at -78°C. Et₃N (4.90 mL, 35.16 mmol) is added dropwise and the reaction mixture was kept at -78 °C for an additional 60 minutes. The reaction mixture was allowed to warm to room temperature and was stirred an additional hour. The reaction mixture is washed with water (50 mL), 2 × 0.5 M HCl_{(aq)} (50 mL) and brine (30 mL). The organic phase is dried over MgSO₄ and evaporated, which yielded the desired compound as a clear liquid. (302 mg, 2.19 mmol, 62%).

### Methyl (Z)-3-(4,5,8,9-tetrahydro-1H-cycloocta[d]imidazol-2-yl)propanoate (31)

4-Oxobutanoate (85%, 856 µL, 6.95 mmol, 1.2 equiv.) was dissolved in MeOH (10 mL) and ammonium acetate (1.07 g, 13.90 mmol, 2.40 equiv.) was added in one portion. After 15 min, (*Z*)-cyclooct-5-ene-1,2-dione (800 mg, 5.79 mmol, 1 equiv.) in MeOH (20 mL) was added in one portion. The reaction was stirred for 2.5 days at RT. After completion, the reaction mixture was diluted with H₂O (250 mL) containing 0.5% HCl_{(aq.)} and the aqueous phase was extracted with 3x DCM (100 mL). The aqueous phase was basified with sat. K₂CO_{3(aq.)}, till pH 9 and the aqueous phase was extracted with 3x DCM (100 mL). The combined organic fractions were dried over MgSO₄, filtered and evaporated, which yielded a white solid. The crude was used without purification used in the next reaction (1.16 g, 4.95 mmol, 85%). ¹H NMR (600 MHz, CDCl₃) δ 5.60 - 5.51 (m, 2H), 3.68 (s, 3H), 2.91 (t, *J* = 6.7 Hz, 2H), 2.88 - 2.82 (m, 4H), 2.71 (t, *J* = 6.7 Hz, 2H), 2.47 (dt, *J* = 8.9, 4.8 Hz, 4H). ¹³C NMR (151 MHz, CDCl₃) δ 174.67, 144.28, 128.78, 52.02, 32.72, 27.20, 26.56, 23.58.

### (Z)-3-(4,5,8,9-Tetrahydro-1H-cycloocta[d]imidazol-2-yl)propanoic acid (32)

In a 5 mL screw neck vial 1M LiOH solution (861 µL, 861 µmol) was added to methyl (*E*)-3-(4,5,8,9-tetrahydro-1*H*-cycloocta[*d*]imidazol-2-yl)propanoate (100 mg, 287 µmol) in 2.5 mL THF/water (3:2 v/v). The suspension was stirred at rt for 3 h. The reaction was quenched with 1M TFA_{(aq.)} and submitted to preparative HPLC to yield (E)-3-(4,5,8,9-Tetrahydro-1H-cycloocta[d]imidazol-2-yl)propanoic acid as a light yellow, amorphous solid. (73 mg, 218 µmol, 76%). ¹H NMR (599.65 MHz, DMSO) δ = 12.52 (s, 1H), 5.57 - 5.48 (m, 2H), 3.03 (t, J = 7.2 Hz, 2H), 2.88 (dt, J = 14.2, 6.4 Hz, 2H), 2.77 (t, J = 7.2 Hz, 2H), 2.65 (ddd, J = 14.4, 6.7, 4.9 Hz, 2H), 2.24 (ddd, J = 11.5, 9.4, 4.5 Hz, 2H), 2.16 (ddd, J = 12.1, 7.4, 4.3 Hz, 2H). ¹³C NMR (150.78 MHz, DMSO) δ = 172.6, 142.67, 134.9, 127.6, 30.5, 29.2, 27.8, 20.8.

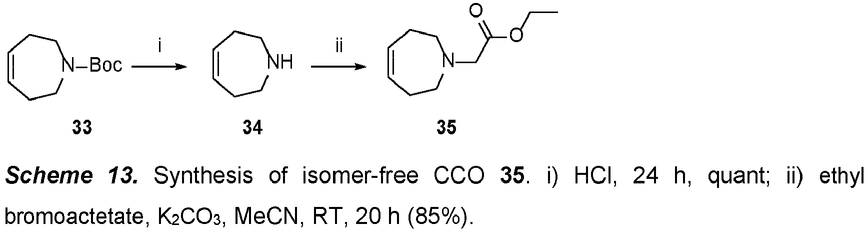

### 2,3,6,7-Tetrahydro-1H-azepine hydrochloride (34)

*tert*-Butyl 2,3,6,7-tetrahydro-1*H*-azepine-1-carboxylate (1.00 g, 5.07 mmol, 1 equiv.) was dissolved in Et₂O (5 mL), containing 2M HCl. The reaction was stirred for 24 h at RT. Upon completion, the solids were filtered off and washed with 3 × Et₂O (5 mL), after the drying the product was obtained as a white solid (676 mg, 5.07 mmol, quant.). ¹H NMR (400 MHz, MeOD) δ 5.91 (t, *J* = 3.5 Hz, 2H), 3.27 - 3.19 (m, 4H), 2.52 (q, *J* = 4.4 Hz, 4H). ¹³C NMR (101 MHz, MeOD) δ 131.3, 46.7, 26.3.

### Ethyl 2-(2,3,6,7-tetrahydro-1H-azepin-1-yl)acetate (35)

2,3,6,7-Tetrahydro-1*H*-azepine hydrochloride (100 mg, 0.75 mmol, 1 equiv.) was dissolved in dry MeCN (4 mL), to which was added K₂CO₃, followed by ethyl bromoactetate (83 µL, 0.75 mmol, 1 equiv.). The reaction was stirred for 20 h at RT. The crude was extracted with 3 × DCM (10 mL). The compound organic fraction were washed with 3 × 2 M K₂CO_{3(aq)} (50 mL) and washed with brine (25 mL) and dried over MgSO₄ and under high vac for 60 min, which yielded the compound at a pale yellow oil (116 mg, 0.63 mmol 85%). ¹H NMR (600 MHz, CDCl₃) δ 5.79 (td, *J* = 3.2, 1.5 Hz, 2H), 4.18 (q, *J* = 7.1 Hz, 2H), 3.38 (s, 2H), 2.78 - 2.69 (m, 4H), 2.27 (q, *J* = 4.3 Hz, 4H), 1.27 (t, *J* = 7.1 Hz, 3H). ¹³C NMR (151 MHz, CDCl₃) δ 171.22, 131.41, 60.63, 59.68, 54.87, 28.98, 14.44.

### Example 4

### Test of different combinations of 1,2,4,5-tetrazines and dienophile with at least one double bond in the cis-configuration s in different solvents and under various oxidative conditions

Figures 2 - 5 show the reaction between eight different 1,2,4,5-tetrazines and four different cis-dienophiles, dissolved in 1:1 H₂O/EtOH (% v/v), DMSO/H₂O (5/95), or MeCN/H₂O (87.5:12.5). The UV-light source applied in the transformation/ligation/oxidation step was either a strong UV-source;(254 or 356 nm, UV irradiance of 3200 mW/cm³); or with a mild UV-source;(254 nm, UV irradiance of 0.5 mW/cm³). The acid applied in the transformation/ligation/oxidation step was either HCl, TFA or H₃PO₄. The transformation/ligation/oxidation step did either not include a photosensitizer, or a photosensitizer selected from Methyl benzoate, Salicylic acid and Benzophenone was included. The figures show the results obtained under these various conditions during the oxidation step are shown as yield (%) of the pyridazine obtained out of the total yield possible from the reaction, measured by HPLC Also, the time to completion (in min) is shown.

## Claims

1. Method for providing pyridazines based on inverse electron demand Diels-Alder cycloaddition reactivity reaction between a tetrazine and a dienophile comprising:
a) Mixing:
a dienophile selected from the group consisting of cyclenes having one or more rings wherein one ring is composed of at least 7 atoms, and having at least one unconjugated double bond in the cis-configuration; said dienophile being conjugated to a labeling agent and/or to a pharmaceutic agent, a diagnostic agent, or a therapeutic agent, or said dienophile being conjugated to a targeting vector; and
a 1,2,4,5-tetrazine being conjugated to a labeling agent and/or to a pharmaceutic agent, a diagnostic agent, or a therapeutic agent, or said 1,2,4,5-tetrazine being conjugated to a targeting vector;
in a solvent comprising at least 1% water,
wherein when said dienophile is conjugated to a labeling agent and/or to a pharmaceutic agent, a diagnostic agent, or a therapeutic agent, then said 1,2,4,5-tetrazine is conjugated to a targeting vector;
and wherein when said dienophile is conjugated to a targeting vector, then said 1,2,4,5-tetrazine is conjugated to a labeling agent and/or a pharmaceutic agent, a diagnostic agent, or a therapeutic agent; and
b) subjecting the mixture obtained from step a) to a one-pot-three-step reaction at a temperature ranging from 15 °C to 50 °C by addition of an acid catalyst having a pKa of 5 or below in H₂O at 25 °C, at a concentration of 0.1 M - 5 M, and exposure to UV-light with a minimum UV irradiance of 0.5 mW/cm³.

2. Method according to claim 1 wherein said dienophile in step a) is selected from a C7 - C12 cyclene, such as a C8 cyclene, and having at least one unconjugated double bond in the *cis*-configuration.

3. Method according to claim 1 or 2, wherein the dienophile is selected from the group comprising: a monocyclic unsaturated hydrocarbon, a bicyclic unsaturated hydrocarbon, a monocyclic unsaturated heterocycle, and a bicyclic unsaturated heterocycle.

4. Method according to any of the previous claims, wherein the labeling agent in step a) is a radionuclide or a stable isotope of a corresponding element such as ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac.

5. Method according to any of the previous claims, further comprising the addition of one or more photosensitizers in step b).

6. Method according to any of the previous claims, wherein a photosensitizer is conjugated to the 1,2,4,5-tetrazine.

7. Method according to any of the previous claims, wherein a photosensitizer is conjugated to the dienophile cyclene having one or more rings wherein one ring is composed of at least 7 atoms, and having at least one unconjugated double bond in the cis-configuration.

8. Method according to any of claims 5 to 7, wherein the photosensitizer is selected from one or more of a benzoate based photosensitizer, a fluorescein based photosensitizer, and a porphyrin based photosensitizer.

9. Method according to any of the previous claims, wherein the dienophile in step a) is an isomer-free cyclene having one or more rings wherein one ring is composed of at least 7 atoms, and having at least one unconjugated double bond in the cis-configuration.

10. Method according to any of the previous claims, wherein the dienophile having one or more rings wherein one ring is composed of at least 7 atoms, and having at least one unconjugated double bond in the cis-configuration selected from: 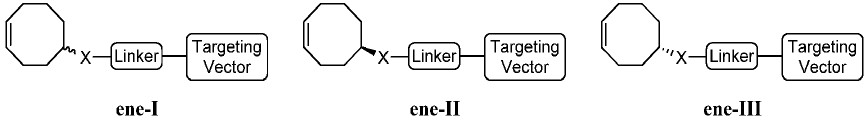 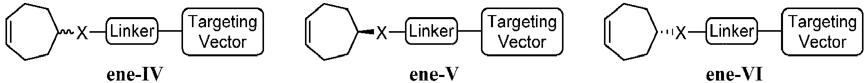 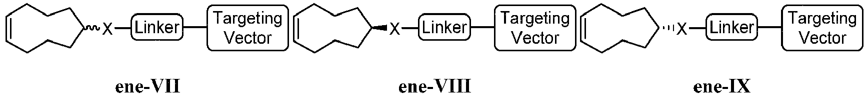 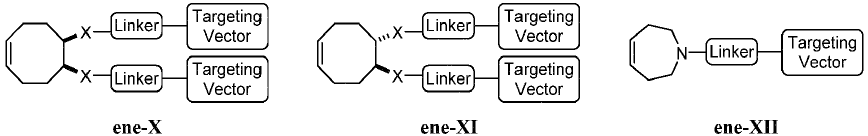 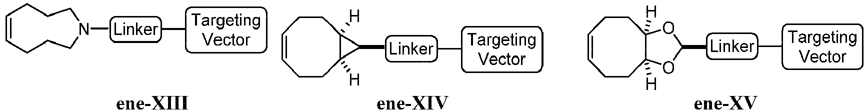 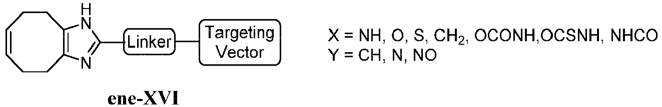
wherein the linker is selected from the group comprising: -(CH₂)ₙ- (CH₂)ₙNH, (CH₂)ₙCO, (CH₂)ₙO, (CH₂CH₂O)ₙ, (CH₂CH₂O)ₙCH₂CH₂NH, (CH₂CH₂O)ₙCH₂CH₂CO, - CO(CH)₂- CO(CH₂)ₙNH, CO(CH₂)ₙCO, CO(CH₂)ₙO, CO(CH₂CH₂O)ₙ CO(CH₂CH₂O)ₙCH₂CH₂NH, CO(CH₂CH₂O)ₙCH₂CH₂CO, COO(CH)₂- COO(CH₂)ₙNH, COO(CH₂)ₙCO, COO(CH₂)ₙO, COO(CH₂CH2O)ₙ COO(CH₂CH₂O)ₙCH₂CH₂NH, COO(CH₂CH₂O)ₙCH₂CH₂CO, CONH(CH)₂-CONH(CH₂)ₙNH, CONH(CH₂)ₙCO, CONH(CH₂)ₙO, CONH(CH₂CH₂O)ₙ, CONH(CH₂CH₂O)ₙCH₂CH₂NH, CONH(CH₂CH₂O)ₙCH₂CH₂CO, -CONHPhCO, -COOPhCO, -COPhCO, CONHCHMCO, (CH₂)ₙNHCHMCO, (CH₂)ₙOCONHCHMCO, (CH₂)ₙNHCHMCO, (CH₂)ₙNHCOCHMNH, (CH₂)OCOCHMNH, (CH₂CH₂O)ₙCH₂CH₂NHCHMCO, (CH₂CH₂O)ₙCH₂CH₂CONHCHMCO, (CH₂CH₂O)ₙCH₂CH₂NHCHMCO, (CH₂CH₂O)ₙCH₂CH₂NHCOCHMNH, (CH₂CH₂O)ₙCOCHMNH, where n is 0-25 and where M is a side chain selected from the group consisting of side chains of the natural amino acids: H, CH₃, CH₂SH, CH₂COOH, CH₂CH₂COOH, CH₂C₆H₅, CH₂C₃H₃N₂, CH(CH₃)CH₂CH₃, (CH₂)₄NH₂, CH₂CH(CH₃)₂, CH₂CH₂SCH₃, CH₂CONH₂, (CH₂)₄NHCOC₄H₅NCH₃, CH₂CH₂CH₂, CH₂CH₂CONH₂, (CH₂)₃NH-C(NH)NH₂, CH₂OH, CH(OH)CH₃, CH₂SeH, CH(CH₃)₂, CH₂C₈H₆N, CH₂C₆H₄OH;
and wherein the targeting vector is an antibody, a nanobody, a polymer, a nanomedicine, a cell, a protein, a peptide, or a small molecule.

11. Method according to any of the previous claims, wherein the 1,2,4,5-tetrazine is of formula Tz1: 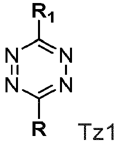
wherein R and R₁ are independently selected from -H, -Me or 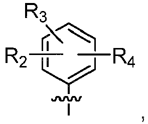 wherein the curly sign indicates the link to the tetrazine; and where R₂ is -H or (i) an isotope labeling agent directly connected to the aromatic ring; or (ii) an isotope labeling agent connected to the aromatic ring via a linker, said linker being selected from the group consisting of -(CH₂)ₙ, LO(CH₂)ₙ, -LNH(CH₂)ₙ, - LCONH(CH₂)ₙ, -LNHCO(CH₂)ₙ, where L is -(CH₂)ₘ or -(CH₂CH₂O)ₘ, where n and m are independently selected from 1-25; or (iii) an isotope labeling agent that is chelated through a chelator selected from: 1,4,7,10-tetraazacyclododecane-*N*,*N*',*N*',*N*"-tetraacetic acid (DOTA), *N*,*N*'-bis(2-hydroxy-5-(carboxyethyl)benzyl)ethylenediamine *N*,*N*'-diacetic acid (HBED-CC), 14,7-triazacyclononane-1,4,7-triacetic acid (NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15), 11,13-triene-3,6,9-triacetic acid (PCTA), *N*'-(5-acetyl (hydroxy)aminopentyl-N-(5-(4-(5-aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA) connected to the aromatic ring through a linker, said linker being selected from the group consisting of -(CH₂)ₙ, -LO(CH₂)ₙ, - LNH(CH₂)ₙ, -LCONH(CH₂)ₙ, -LNHCO(CH₂)ₙ, where L is -(CH₂)ₘ or --(CH₂CH₂O)ₘ, and n and m are independently selected from 1-25;
wherein, when R₂ is either (i) or (ii) the isotope labeling agent is selected from the group consisting of:
¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ⁵⁸mCo, ⁵⁹Co, ⁶⁰Cu, ⁶¹Cu, ⁶³Cu, ⁶⁴Cu, ⁶⁵Cu, ⁶⁷Cu_{,} ⁶⁷Ga, ⁶⁸Ga, ⁶⁹Ga, ⁷¹Ga, ⁷⁶Br, ⁷⁷Br, ⁷⁹Br, ⁸⁰mBr, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh, ¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²¹¹At, ²²³Ra, ²²⁵Ac,
and wherein R₃ and R₄ are independently selected from H or a moiety selected from the group consisting of a hydroxy group, a sulfonamide, a guanidyl group, a carboxyl group, a sulfonyl group, amine, a substituted amine with 1-25 polyethylene glycol unit(s), a -(O-CH₂-CH₂)ₙ--OCH₂-COOH, and n is selected from 1-25; or Methyl, Ethyl, Propyl, optionally substituted heteroaryl, and optionally substituted arylalkyl; wherein optionally substituted in relation to said substituted amine means one or more substituents selected from, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-25 polyethylene glycol unit(s), a -(O-CH₂-CH₂)ₙ-OCH₂-COOH, and n is selected from 1-25; or H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, and amine;

12. Method according to any of the previous claims, wherein the 1,2,4,5-tetrazine is selected from:

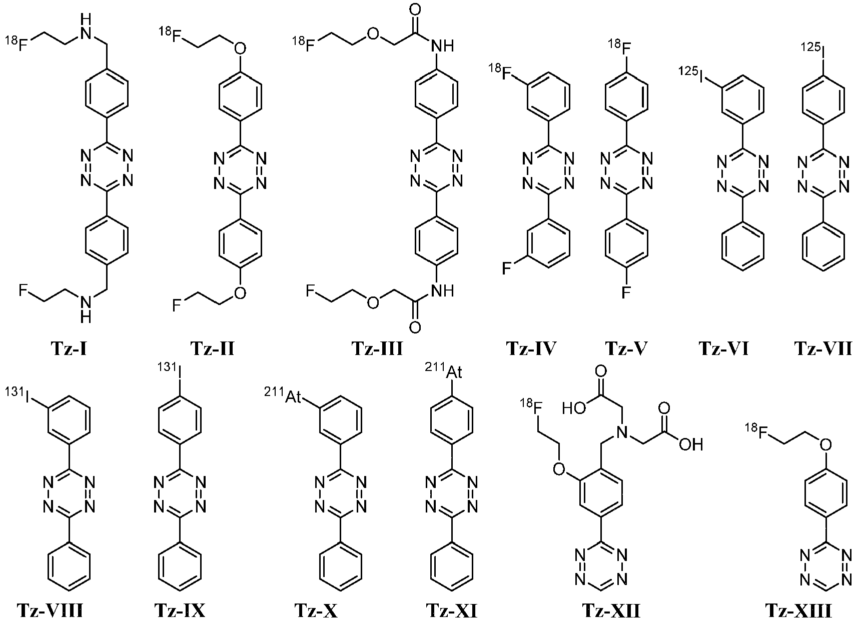

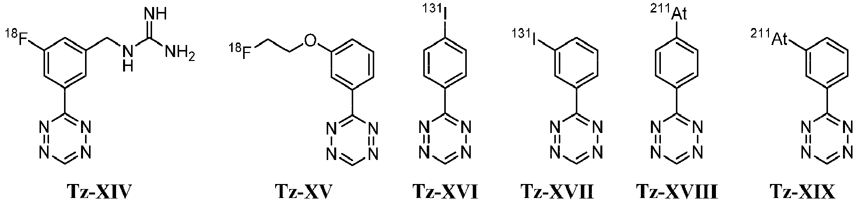

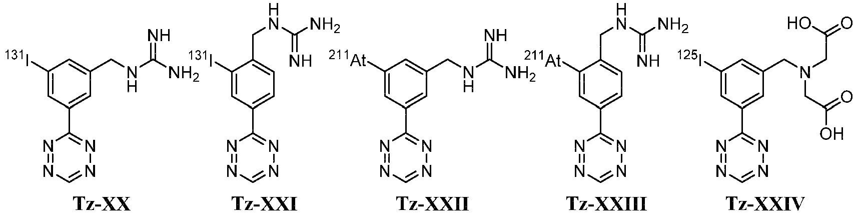

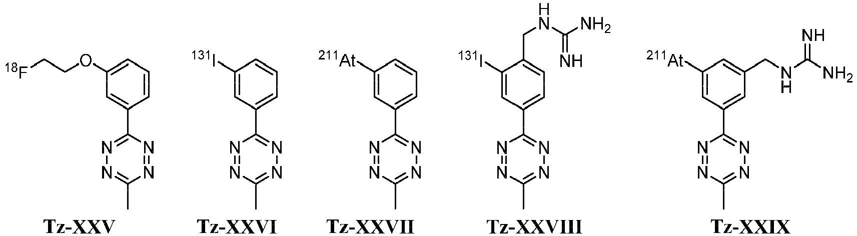

13. Method according to any of the previous claims, wherein the 1,2,4,5-tetrazine comprises a photosentizier and is selected from:

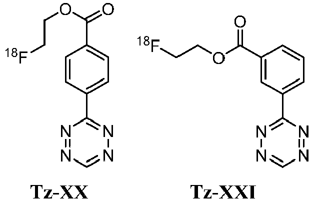

14. Method according to any of claims 1 - 10, wherein the 1,2,4,5-tetrazine is symmetrical substituted tetrazine wherein at least one of the symmetry planes pass through the nitrogen-nitrogen bonds of at least one tetrazine ring.

15. Method according to claim 14, wherein the 1,2,4,5-symmetrical tetrazine is selected from:
